# EUROPEAN PATENT APPLICATION

(11) **EP 3 533 459 A1**
(43) Date of publication of application: **04.09.2019**
(21) Application number: 18305228.1
(22) Date of filing: 02.03.2018
(51) Int. Cl.: A61K 39/00, C07K 16/40

(54) **ANTI-PLA2-GIB ANTIBODIES AND THE USES THEREOF**

(71) Applicant: Diaccurate, 75008 Paris (FR)
(72) Inventor: TAMARIT, Blanche, 75014 Paris (FR); THÈZE, Jacques, 75007 Paris (FR)
(74) Representative: Cabinet Becker et Associés

(57) **Abstract**

The present invention relates to anti-PLA2-GIB antibodies and the uses thereof, particularly for detecting human PLA2-GIB in a sample.

SEQUENCE LISTING

<110>  DIACCURATE

<120>  ANTI-PLA2-GIB ANTIBODIES AND THE USES THEREOF

<130>  B2700

<160>  26

<170>  PatentIn version 3.5

<210>  1
<211>  148
<212>  PRT
<213>  Homo sapiens

<400>  1

Met Lys Leu Leu Val Leu Ala Val Leu Leu Thr Val Ala Ala Ala Asp
1               5                   10                  15

Ser Gly Ile Ser Pro Arg Ala Val Trp Gln Phe Arg Lys Met Ile Lys
            20                  25                  30

Cys Val Ile Pro Gly Ser Asp Pro Phe Leu Glu Tyr Asn Asn Tyr Gly
        35                  40                  45

Cys Tyr Cys Gly Leu Gly Gly Ser Gly Thr Pro Val Asp Glu Leu Asp
    50                  55                  60

Lys Cys Cys Gln Thr His Asp Asn Cys Tyr Asp Gln Ala Lys Lys Leu
65                  70                  75                  80

Asp Ser Cys Lys Phe Leu Leu Asp Asn Pro Tyr Thr His Thr Tyr Ser
                85                  90                  95

Tyr Ser Cys Ser Gly Ser Ala Ile Thr Cys Ser Ser Lys Asn Lys Glu
            100                 105                 110

Cys Glu Ala Phe Ile Cys Asn Cys Asp Arg Asn Ala Ala Ile Cys Phe
        115                 120                 125

Ser Lys Ala Pro Tyr Asn Lys Ala His Lys Asn Leu Asp Thr Lys Lys
    130                 135                 140

Tyr Cys Gln Ser
145


<210>  2
<211>  117
<212>  PRT
<213>  Artificial Sequence

<220>
<223>  14G9 VL

<400>  2

Asp Ile Val Met Thr Gln Ser His Lys Leu Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Glu Lys Pro Gly Gln Ser Pro Lys Leu Leu Ile
        35                  40                  45

Tyr Trp Ala Ser Thr Arg His Thr Gly Val Pro Asn Arg Phe Thr Gly
    50                  55                  60

Ser Arg Ser Gly Thr Asp Tyr Thr Leu Thr Ile Ser Ser Val Gln Ala
65                  70                  75                  80

Glu Asp Leu Ala Leu Tyr Tyr Cys Gln Gln Asp Tyr Ser Thr Pro Pro
                85                  90                  95

Thr Phe Gly Ala Gly Thr Lys Leu Glu Leu Lys Arg Ala Asp Ala Ala
            100                 105                 110

Pro Thr Val Ser Ile
        115


<210>  3
<211>  351
<212>  DNA
<213>  Artificial Sequence

<220>
<223>  14G9 VL

<400>  3
gacattgtga tgacccagtc tcacaaactc atgtccacat cagtaggaga cagggtcagc      60

atcacctgca aggccagtca ggatgtgagt actgctgtag cctggtatca agaaaaacca     120

ggtcaatctc ctaaactact gatttactgg gcatccaccc ggcacactgg agtccctaat     180

cgcttcacag gcagtagatc tgggacagat tatactctca ccatcagcag tgtgcaggct     240

gaagacctgg cactttatta ctgtcagcaa gattatagca ctcctcctac gttcggtgct     300

gggaccaagc tggagctgaa acgggctgat gctgcaccaa ctgtatccat c              351


<210>  4
<211>  130
<212>  PRT

<213>  Artificial Sequence

<220>
<223>  14G9 VH

<400>  4

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Asn Tyr
            20                  25                  30

Trp Ile His Trp Val Lys Arg Arg Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Lys Ala Thr Leu Ala Val Asp Lys Ser Ser Ser Thr Ala Tyr
65                  70                  75                  80

Met Gln Leu Ser Ser Leu Thr Ser Glu Asp Ser Ala Val Phe Tyr Cys
                85                  90                  95

Ala Arg Gln Thr Leu Tyr Tyr Glu Ala Leu Asp Tyr Trp Gly Gln Gly
            100                 105                 110

Thr Thr Leu Thr Val Ser Ser Ala Lys Thr Thr Pro Pro Ser Val Tyr
        115                 120                 125

Pro Leu
    130

<210>  5
<211>  392
<212>  DNA
<213>  Artificial Sequence

<220>
<223>  14G9 VH

<400>  5
caggtccaac tgcaacagcc tggggctgag ctggtgaagc ctggggcttc agtgaagctg      60

tcctgcaagg cttctggcta caccttcacc aactactgga tacactgggt gaagcggagg     120

cctggacaag gccttgaatg gattggtaat attgaccctt ctgatactag aactcactac     180

aatcaaaagt tcaaggacaa ggccacattg gctgtagaca aatcctccag cacagcctac     240

atgcagctca gcagcctgac atctgaggac tctgcggtct tttactgtgc aagacagacc     300

ctctactatg aggccttgga ctactggggc caaggcacca ctctcacagt ctcctcagcc     360

aaaacgacac ccccatctgt ctatccactg gg                                392

<210>  6
<211>  366
<212>  DNA
<213>  Artificial Sequence

<220>
<223>  22C6-VL

<400>  6
gaaattgttc tcacccagtc tccaaccacc atggctgcat ctcccgggga gaagatcact      60

atcacctgca gcgtcagctc aagtataagt cccaatttct tgcattggta tcagcagaag     120

ccaggattct ccccgaaact cttgattttt aggacatcca atctggcttc tggagtccca     180

actcgcttca gtggcagtgg gtctgggacc tcttactctc tcacaattgg caccatggag     240

gctgaagacg ttgccactta ttactgtcac cagggtaata gtttaccgct cacgttcggt     300

gctgggacca agctggagct gaaacgggct gatgctgcac caactgtatc catcttccca     360

ccatcc                                                                366

<210>  7
<211>  121
<212>  PRT
<213>  Artificial Sequence

<220>
<223>  22C6-VL

<400>  7

Glu Ile Val Leu Thr Gln Ser Pro Thr Thr Met Ala Ala Ser Pro Gly
1               5                   10                  15

Glu Lys Ile Thr Ile Thr Cys Ser Val Ser Ser Ser Ile Ser Pro Asn
            20                  25                  30

Phe Leu His Trp Tyr Gln Gln Lys Pro Gly Phe Ser Pro Lys Leu Leu
        35                  40                  45

Ile Phe Arg Thr Ser Asn Leu Ala Ser Gly Val Pro Thr Arg Phe Ser
    50                  55                  60

Gly Ser Gly Ser Gly Thr Ser Tyr Ser Leu Thr Ile Gly Thr Met Glu
65                  70                  75                  80

Ala Glu Asp Val Ala Thr Tyr Tyr Cys His Gln Gly Asn Ser Leu Pro
                85                  90                  95

Leu Thr Phe Gly Ala Gly Thr Lys Leu Glu Leu Lys Arg Ala Asp Ala

            100                 105                 110

Ala Pro Thr Val Ser Ile Phe Pro Pro
        115                 120

<210> 8
<211> 453
<212> DNA
<213> Artificial Sequence

<220>
<223> 22C6-VH

<400> 8
gaggtgcagc tgcaggagtc aggacctggc ctggtgaagc cttctcagac agtgtccctc 60

aactgcactg tcactggcca ctctatcact gatgataatt actattggag ctggatccgg 120

ctggtttcag gaagcaaact ggagtggata gggtacataa cctccgctgg taacactgac 180

aacaatcctt ctctcaaaag tcgcatctcc atcacccggg acatttccaa gaaccaattc 240

ttcctgcact tgagctctgt gactgctgaa gatacagcca catattactg tgcaagagat 300

tcaactgatg gtgactttta ctttgaaaaa tggggccaag gcaccactct cacagtctcc 360

tcagccaaaa caacagcccc atcggtctat ccactggccc ctgtgtgtgg agatacaagt 420

ggctcctcgg tgactctagg atgcctggtc aag 453

<210> 9
<211> 151
<212> PRT
<213> Artificial Sequence

<220>
<223> 22C6-VH

<400> 9

Glu Val Gln Leu Gln Glu Ser Gly Pro Gly Leu Val Lys Pro Ser Gln
1               5                   10                  15

Thr Val Ser Leu Asn Cys Thr Val Thr Gly His Ser Ile Thr Asp Asp
            20                  25                  30

Asn Tyr Tyr Trp Ser Trp Ile Arg Leu Val Ser Gly Ser Lys Leu Glu
        35                  40                  45

Trp Ile Gly Tyr Ile Thr Ser Ala Gly Asn Thr Asp Asn Asn Pro Ser
    50                  55                  60

Leu Lys Ser Arg Ile Ser Ile Thr Arg Asp Ile Ser Lys Asn Gln Phe
65                  70                  75                  80

Phe Leu His Leu Ser Ser Val Thr Ala Glu Asp Thr Ala Thr Tyr Tyr
                85                  90                  95

Cys Ala Arg Asp Ser Thr Asp Gly Asp Phe Tyr Phe Glu Lys Trp Gly
            100                 105                 110

Gln Gly Thr Thr Leu Thr Val Ser Ser Ala Lys Thr Thr Ala Pro Ser
        115                 120                 125

Val Tyr Pro Leu Ala Pro Val Cys Gly Asp Thr Ser Gly Ser Ser Val
    130                 135                 140

Thr Leu Gly Cys Leu Val Lys
145                 150

<210>  10
<211>  339
<212>  DNA
<213>  Artificial Sequence

<220>
<223>  24B2-VL

<400>  10
gacattgtgc tgacccaatc tccagcttcc ttggctgtgt ctctagggca gagggccacc      60

atctcttgca gagccagcga aagtgttgat aattatggca ttagttttat gcactggtac     120

cagcagaaac cgggacagcc acccaaactc ctcatctatc gtgcatccaa cctagaatct     180

gggatccctg ccaggttcag tggcagtggg tctaggacag acttcaccct caccattaat     240

cctgtggaga ctgatgatgt tgcaacctat tattgtcagc aaagtagtag ggatctgttc     300

acgttcggag gggggactaa gctggaaata aaacgggcg                            339

<210>  11
<211>  113
<212>  PRT
<213>  Artificial Sequence

<220>
<223>  24B2-VL

<400>  11

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Asn Tyr
            20                  25                  30

Gly Ile Ser Phe Met His Trp Tyr Gln Gln Lys Pro Gly Gln Pro Pro
        35                  40                  45

Lys Leu Leu Ile Tyr Arg Ala Ser Asn Leu Glu Ser Gly Ile Pro Ala
    50                  55                  60

Arg Phe Ser Gly Ser Gly Ser Arg Thr Asp Phe Thr Leu Thr Ile Asn
65                  70                  75                  80

Pro Val Glu Thr Asp Asp Val Ala Thr Tyr Tyr Cys Gln Gln Ser Ser
                85                  90                  95

Arg Asp Leu Phe Thr Phe Gly Gly Gly Thr Lys Leu Glu Ile Lys Arg
            100                 105                 110

Ala

<210>  12
<211>  417
<212>  DNA
<213>  Artificial Sequence

<220>
<223>  24B2-VH

<400>  12
gatgtacaag tgcaggagtc aggacctggc ctggtgaagc cttctcagac agtgtccctc      60

aactgcactg tcactggcca ctctatcact gatgataatt actattggag ctggatccgg     120

ctggtttctg gaagcaaatt ggagtggatc gggtacataa cctccagtgg tagcactgac     180

atcaatcctt ctctcaaaag tcgcatctcc atcacccggg acatttccaa gaaccagttc     240

ttcctgcact tgaactctgt gactgctgaa gatacagcca catattactg tgcaagagat     300

tcaactgatg gtgactttta ctttgaaaaa tggggccaag gcaccactct cacagtctcc     360

tcagccaaaa caacagcccc atcggtctat ccactggccc ctgtgtgtgg agataca        417

<210>  13
<211>  139
<212>  PRT
<213>  Artificial Sequence

<220>
<223>  24B2-VH

<400>  13

Asp Val Gln Val Gln Glu Ser Gly Pro Gly Leu Val Lys Pro Ser Gln
1               5                   10                  15

Thr Val Ser Leu Asn Cys Thr Val Thr Gly His Ser Ile Thr Asp Asp
            20                  25                  30

Asn Tyr Tyr Trp Ser Trp Ile Arg Leu Val Ser Gly Ser Lys Leu Glu
        35                  40                  45

Trp Ile Gly Tyr Ile Thr Ser Ser Gly Ser Thr Asp Ile Asn Pro Ser
    50                  55                  60

Leu Lys Ser Arg Ile Ser Ile Thr Arg Asp Ile Ser Lys Asn Gln Phe
65                  70                  75                  80

Phe Leu His Leu Asn Ser Val Thr Ala Glu Asp Thr Ala Thr Tyr Tyr
                85                  90                  95

Cys Ala Arg Asp Ser Thr Asp Gly Asp Phe Tyr Phe Glu Lys Trp Gly
            100                 105                 110

Gln Gly Thr Thr Leu Thr Val Ser Ser Ala Lys Thr Thr Ala Pro Ser
        115                 120                 125

Val Tyr Pro Leu Ala Pro Val Cys Gly Asp Thr
    130                 135

<210>  14
<211>  330
<212>  DNA
<213>  Artificial Sequence

<220>
<223>  8G11-VL

<400>  14
gacattgtgc tgacacagtc tcctgcttcc ttagctgtat ctctggggca gagggccacc      60

atctcataca gggccagcaa aagtgtcagt acatctggct atagttatat gcactggaac     120

caacagaaac caggacagcc acccagactc ctcatctatc ttgtatccaa cctagaatct     180

ggggtccctg ccaggttcag tggcagtggg tctgggacag acttcaccct caacatccat     240

cctgtggagg aggaggatgc tgcaacctat tactgtcagc acattaggga gcttacacgt     300

tcggaggggg gaccaagctg gaaataaaac                                      330

<210>  15
<211>  108
<212>  PRT
<213>  Artificial Sequence

<220>
<223>  8G11-VL

<400>  15

Asp Ile Val Leu Thr Gln Ser Pro Ala Ser Leu Ala Val Ser Leu Gly
1               5                   10                  15

Gln Arg Ala Thr Ile Ser Tyr Arg Ala Ser Lys Ser Val Ser Thr Ser
            20                  25                  30

Gly Tyr Ser Tyr Met His Trp Asn Gln Gln Lys Pro Gly Gln Pro Pro
        35                  40                  45

Arg Leu Leu Ile Tyr Leu Val Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln His Ile Arg
                85                  90                  95

Glu Leu Thr Arg Ser Glu Gly Gly Pro Ser Trp Lys
            100                 105

<210>  16
<211>  426
<212>  DNA
<213>  Artificial Sequence

<220>
<223>  8G11-VH

<400>  16
caggttcaac tgcagcagtc tggggctgag ctggtgaggc ctggggcttc agtgaagctg      60

tcctgcaagg cttcgggcta cacatttaat gacgatgaaa tgcactgggt gaagcagaca     120

cctgtgcatg gcctggaatg gattggagct attgatcctg aaactggtgg cactgcctac     180

aatcagaagt tcaaggacaa ggccacactg actgcagaca aatcctccag cacagcctac     240

atggagatcc gcagcctgac atctgaggac tctgccgtct attactgtca gttaatcttc     300

catggtaacc cttactattg tatggactac tggggtcaag gaacctcagt caccgtctcc     360

tcagccaaaa caacagcccc atcggtctat ccactggccc ctgtgtgtgg agatacaagt     420

ggctcc                                                                426

<210>  17
<211>  142
<212>  PRT
<213>  Artificial Sequence

<220>
<223>  8G11-VH

<400>  17

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Asn Asp Asp
            20                  25                  30

Glu Met His Trp Val Lys Gln Thr Pro Val His Gly Leu Glu Trp Ile
        35                  40                  45

Gly Ala Ile Asp Pro Glu Thr Gly Gly Thr Ala Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Glu Ile Arg Ser Leu Thr Ser Glu Asp Ser Ala Val Tyr Tyr Cys
                85                  90                  95

Gln Leu Ile Phe His Gly Asn Pro Tyr Tyr Cys Met Asp Tyr Trp Gly
            100                 105                 110

Gln Gly Thr Ser Val Thr Val Ser Ser Ala Lys Thr Thr Ala Pro Ser
        115                 120                 125

Val Tyr Pro Leu Ala Pro Val Cys Gly Asp Thr Ser Gly Ser
    130                 135                 140

<210>  18
<211>  363
<212>  DNA
<213>  Artificial Sequence

<220>
<223>  1C11-VL

<400>  18
gaaattgtgc tcactcagtc tccagccatc acatctgcat ctctggggca aagggtcacc      60

atctcctgca gtgccacctc aagtgtaagt tacgtccact ggtaccagca gaagtcaggc     120

acctccccca aaccatggat ttatgaaata tccagactgg cttctggagt cccagctcgc     180

ttcagtggca gtggatctgg gacctcttac tctctcacaa tcagcaccat ggaggctgaa     240

gatgctgcca tttattactg ccagtcgtgg aattttccac tgctctcgtt cggtgctggg     300

accaagctgg agctgaaacg ggctgatgct gcaccaactg tatccatctt cccaaccatc     360

caa                                                                   363

<210>  19
<211>  121
<212>  PRT
<213>  Artificial Sequence

<220>
<223>  1C11-VL

<400>  19

Glu Ile Val Leu Thr Gln Ser Pro Ala Ile Thr Ser Ala Ser Leu Gly
1               5                   10                  15

Gln Arg Val Thr Ile Ser Cys Ser Ala Thr Ser Ser Val Ser Tyr Val
            20                  25                  30

His Trp Tyr Gln Gln Lys Ser Gly Thr Ser Pro Lys Pro Trp Ile Tyr
        35                  40                  45

Glu Ile Ser Arg Leu Ala Ser Gly Val Pro Ala Arg Phe Ser Gly Ser
    50                  55                  60

Gly Ser Gly Thr Ser Tyr Ser Leu Thr Ile Ser Thr Met Glu Ala Glu
65                  70                  75                  80

Asp Ala Ala Ile Tyr Tyr Cys Gln Ser Trp Asn Phe Pro Leu Leu Ser
                85                  90                  95

Phe Gly Ala Gly Thr Lys Leu Glu Leu Lys Arg Ala Asp Ala Ala Pro
            100                 105                 110

Thr Val Ser Ile Phe Pro Thr Ile Gln
        115                 120


<210>  20
<211>  381
<212>  DNA
<213>  Artificial Sequence

<220>
<223>  1C11-VH

<400>  20
gaggtgaagc tgcaggagtc aggacctgag ctggtgaagc ctggggcctc agtgaagatt      60

tcctgcaagg tttctggcta cgcattcagt agcttctgga tgaactgggt gaagcagagg     120

cctggaaagg gtcttgagtg gattggacgg atttatcctg gagatggaga tattaattac     180

aatgggaagt tcaagggcaa ggccacactg accgcagaca aatcctccag cacagcctac     240

atgcaactca gcagcctgac atctgaggac tctgcggtct acttctgtgt tagaggatgg     300

gcctggtttc cttactgggg ccaggggact ctagtcactg tctctgcagc caaaacgaca     360

cccccatctg tctatccact g                                               381


<210>  21
<211>  127

<212>  PRT
<213>  Artificial Sequence

<220>
<223>  1C11-VH

<400>  21

Glu Val Lys Leu Gln Glu Ser Gly Pro Glu Leu Val Lys Pro Gly Ala
1               5                   10                  15

Ser Val Lys Ile Ser Cys Lys Val Ser Gly Tyr Ala Phe Ser Ser Phe
            20                  25                  30

Trp Met Asn Trp Val Lys Gln Arg Pro Gly Lys Gly Leu Glu Trp Ile
        35                  40                  45

Gly Arg Ile Tyr Pro Gly Asp Gly Asp Ile Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Val Arg Gly Trp Ala Trp Phe Pro Tyr Trp Gly Gln Gly Thr Leu Val
            100                 105                 110

Thr Val Ser Ala Ala Lys Thr Thr Pro Pro Ser Val Tyr Pro Leu
        115                 120                 125


<210>  22
<211>  366
<212>  DNA
<213>  Artificial Sequence

<220>
<223>  7A10-VL

<400>  22
gacatccaaa tgaaccagtc tccatccagt ctgtctgcat ccctcggaga cacaattacc      60

atcacttgcc gtgccagtca gggcattagt atttggttag actggtacca gcagaaacca     120

ggaaatattc ctaaactatt gatctataag gcttccaact tgcacacagg agtcccacca     180

aggtttagtg gcagtggatc tggaacagat ttcacattaa ctatcagcag cctacagcct     240

gaagacattg ccacttacta ctgtctacag agtcaaagtt atcctctcac gttcggtgct     300

gggaccaagc tggagctgaa acgggctgat gctgcaccaa ctgtatccat cttcccacca     360

tccaaa                                                                366

<210>  23
<211>  122
<212>  PRT
<213>  Artificial Sequence

<220>
<223>  7A10-VL

<400>  23

Asp Ile Gln Met Asn Gln Ser Pro Ser Ser Leu Ser Ala Ser Leu Gly
1               5                   10                  15

Asp Thr Ile Thr Ile Thr Cys Arg Ala Ser Gln Gly Ile Ser Ile Trp
            20                  25                  30

Leu Asp Trp Tyr Gln Gln Lys Pro Gly Asn Ile Pro Lys Leu Leu Ile
        35                  40                  45

Tyr Lys Ala Ser Asn Leu His Thr Gly Val Pro Pro Arg Phe Ser Gly
    50                  55                  60

Ser Gly Ser Gly Thr Asp Phe Thr Leu Thr Ile Ser Ser Leu Gln Pro
65                  70                  75                  80

Glu Asp Ile Ala Thr Tyr Tyr Cys Leu Gln Ser Gln Ser Tyr Pro Leu
                85                  90                  95

Thr Phe Gly Ala Gly Thr Lys Leu Glu Leu Lys Arg Ala Asp Ala Ala
            100                 105                 110

Pro Thr Val Ser Ile Phe Pro Pro Ser Lys
        115                 120


<210>  24
<211>  396
<212>  DNA
<213>  Artificial Sequence

<220>
<223>  7A10-VH

<400>  24
caggtgcagc tgaaggagtc aggacctggc ctggtggcgc cctcacagag cctgtccatc      60

acatgcactg tctcagggtt ctcattaacc agctatggtg taagctgggt tcgccagcct     120

ccaggaaagg gtctggagtg gctgggagta atatggggtg acgggagcac aaattatcat     180

tcagctctca tatccagact gagcatcagc aaggataact ccaagagcca agttttctta     240

aaactgaaca gtctgcaaac tgatgacaca gccacgtact actgtgccaa agaaggagga     300

ttacgacgcg gggtctactt tgactactgg ggccaaggca ccactctcac agtctcctca     360

gccaaaacga cacccccatc tgtcaattcc actgaa                                396

<210>  25
<211>  132
<212>  PRT
<213>  Artificial Sequence

<220>
<223>  7A10-VH

<400>  25

Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Thr Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Ser Thr Asn Tyr His Ser Ala Leu Ile
    50                  55                  60

Ser Arg Leu Ser Ile Ser Lys Asp Asn Ser Lys Ser Gln Val Phe Leu
65                  70                  75                  80

Lys Leu Asn Ser Leu Gln Thr Asp Asp Thr Ala Thr Tyr Tyr Cys Ala
                85                  90                  95

Lys Glu Gly Gly Leu Arg Arg Gly Val Tyr Phe Asp Tyr Trp Gly Gln
            100                 105                 110

Gly Thr Thr Leu Thr Val Ser Ser Ala Lys Thr Thr Pro Pro Ser Val
        115                 120                 125

Asn Ser Thr Glu
    130

<210>  26
<211>  126
<212>  PRT
<213>  Homo sapiens

<400>  26

Ala Val Trp Gln Phe Arg Lys Met Ile Lys Cys Val Ile Pro Gly Ser
1               5                   10                  15

Asp Pro Phe Leu Glu Tyr Asn Asn Tyr Gly Cys Tyr Cys Gly Leu Gly
            20                  25                  30

Gly Ser Gly Thr Pro Val Asp Glu Leu Asp Lys Cys Cys Gln Thr His
        35                  40                  45

Asp Asn Cys Tyr Asp Gln Ala Lys Lys Leu Asp Ser Cys Lys Phe Leu
    50                  55                  60

Leu Asp Asn Pro Tyr Thr His Thr Tyr Ser Tyr Ser Cys Ser Gly Ser
65                  70                  75                  80

Ala Ile Thr Cys Ser Ser Lys Asn Lys Glu Cys Glu Ala Phe Ile Cys
                85                  90                  95

Asn Cys Asp Arg Asn Ala Ala Ile Cys Phe Ser Lys Ala Pro Tyr Asn
            100                 105                 110

Lys Ala His Lys Asn Leu Asp Thr Lys Lys Tyr Cys Gln Ser
        115                 120                 125

## Description

The present invention relates to anti-PLA2-GIB antibodies and the uses thereof, particularly for detecting human PLA2-GIB in a sample. The invention more particularly discloses new monoclonal antibodies which can discriminate the mature secreted form and the pro form of PLA2-GIB, or which can bind both PLA2-GIB forms. The invention also provides methods for detecting or dosing PLA2-GIB in a sample using such antibodies or fragments thereof, as well as methods for discriminating the pro and secreted forms thereof and kits suitable to perform said methods.

### INTRODUCTION

The group IB pancreatic phospholipase A2 (PLA2-GIB) is a low molecular weight (14 kD), highly stable (7 disulfide bonds), secreted protein, that catalyzes the hydrolysis of the sn-2 fatty acyl bond of phospholipids to release free fatty acids and lysophospholipids (Lambeau & Gelb 2008). In human, the PLA2-GIB gene is mainly expressed in the pancreas, with weaker expression levels in the duodenum, jejunum, and stomach (Eskola et al. 1983a) (Nevalainen & Haapanen 1993). Marginal expression has been described in other tissues, such as the lung, eyes (Kolko et al. 2007) and testis. In human pancreas, PLA2-GIB is mostly synthesized in apical zymogen granule portion of pancreatic acinar cells (Eskola et al. 1983a). PLA2-GIB has also been detected in insulin secretory granules of pancreatic islet β-cells and to be co-secreted with insulin from glucose-stimulated islets (Ramanadham et al. 1998).

PLA2-GIB is first secreted in the pancreatic juice as an inactive pro form (pro-sPLA2-GIB), which is activated by proteolytic cleavage of a pro-peptide, leading to the active mature secreted form (sPLA2-GIB). In vitro experiments suggested that trypsin and plasmin are able to activate pro-sPLA2-GIB with good potency (Nakano et al. 1994).
In addition to a role in the gastrointestinal tract, PLA2-GIB is also circulating in human plasma ((Nishijima et al. 1983) (Eskola et al. 1983b) (Stemby 1984) (Nevalainen et al. 1985) (Kitagawa et al. 1991)). The inventors have previously characterized sPLA2-GIB as a key endogenous factor of the immune response. They demonstrated that sPLA2-GIB plays a crucial role in the mechanism underlying the unresponsiveness of CD4 T lymphocytes observed for example in viremic HIV-infected patient.

There is therefore a need for methods allowing an accurate and sensitive detection of PLA2-GIB. There is also a need for methods and kits that can discriminate the pro and the mature secreted forms of PLA2-GIB.

### SUMMARY OF THE INVENTION

The present invention provides novel anti-PLA2-GIB antibodies and the uses thereof. The invention also provides methods for detecting or dosing PLA2-GIB using such antibodies, as well as kits comprising the same.

In a first aspect, the present invention more specifically relates to monoclonal antibodies, or fragments thereof, that bind to human sPLA2-GIB protein and competitively inhibit the binding to human sPLA2-GIB of a monoclonal antibody comprising a light chain variable region comprising SEQ ID NO: 2 and a heavy chain variable region comprising SEQ ID NO: 4 (14G9).

In another aspect, the present invention relates to monoclonal antibodies or fragments thereof that bind to an epitope on human sPLA2-GIB protein, wherein said epitope comprises at least one amino acid residue selected from W3, R6, K7, K10, C77, Y75, G79 and S80 of human sPLA2-GIB protein, by reference to SEQ ID NO: 26.

In a further aspect, the present invention provides monoclonal antibodies, or fragments thereof, that bind to human pro-sPLA2-GIB protein and competitively inhibit the binding to human pro-sPLA2-GIB of a monoclonal antibody comprising a light chain variable region comprising SEQ ID NO: 15 and a heavy chain variable region comprising SEQ ID NO: 17 (8G11).

In another aspect, the present invention relates to monoclonal antibodies, or fragments thereof, that bind both the mature secreted form (sPLA2-GIB) and the pro- form (pro-sPLA2-GIB) of human PLA2-GIB protein. In a particular embodiment, such antibodies or fragments competitively inhibit the binding to the pro and/or mature secreted form of human PLA2-GIB protein of (i) a monoclonal antibody comprising a light chain variable region comprising SEQ ID NO: 19 and a heavy chain variable region comprising SEQ ID NO: 21 (1C11) or of a monoclonal antibody comprising a light chain variable region comprising SEQ ID NO: 23 and a heavy chain variable region comprising SEQ ID NO: 25 (7A10).

Specific examples of antibodies of the invention include, for instance, 14G9, 24B2, 22C6, 28A1, 28A3, 34G3, 22H5, 7C5, 8G11, 18D9, 1C11, 4G11, 7H12, 7E2, 7A10, 36G10, 3B2, 18A6 and 13H8, as well as fragments thereof.

The invention further relates to an isolated nucleic acid encoding an antibody or fragment of the invention, as well as to a vector comprising such a nucleic acid, preferably operably linked to a promoter.

The invention further relates to a host cell comprising a nucleic acid or a vector as defined above.

In a further aspect, the present invention relates to a method for producing an antibody of the invention, comprising culturing a host cell as defined above under condition suitable for expression of the antibody, and optionally recovering said antibody.

In another aspect, the present invention relates to a composition comprising an antibody, nucleic acid, vector or host cell as defined above.

In another aspect, the present invention relates to an antibody or a composition as defined above for use in the diagnosis, prevention or treatment of a pathological condition in a subject, particularly a condition associated with an activity of PLA2-GIB.

In a further aspect, the present invention relates to a method for detecting human PLA2-GIB protein using an antibody or fragment as defined above. The method can be used or implemented for detecting the presence of the mature secreted form (sPLA2-GIB), the pro- form (pro-sPLA2-GIB) and/or both forms of the human PLA2-GIB protein. Such method or antibodies may be used to detect or dose (or quantify) the presence or amount of PLA2-GIB in a sample, as well as to assess the ratio of pro and mature secreted forms thereof. The methods are particularly useful for detecting or monitoring pathological conditions in subjects that are associated with an activity of PLA2-GIB.

In a further aspect, the present invention relates to kits comprising a least one antibody, or fragment thereof, as herein described and a reagent to perform, detect or quantify an immune reaction, or an antibody-antigen complex.

### BRIEF DESCRIPTION OF THE DRAWINGS

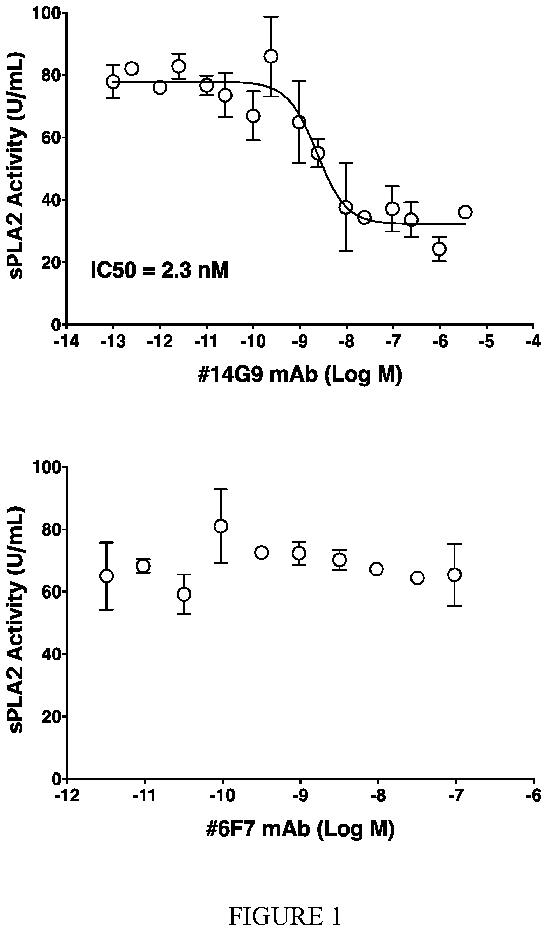

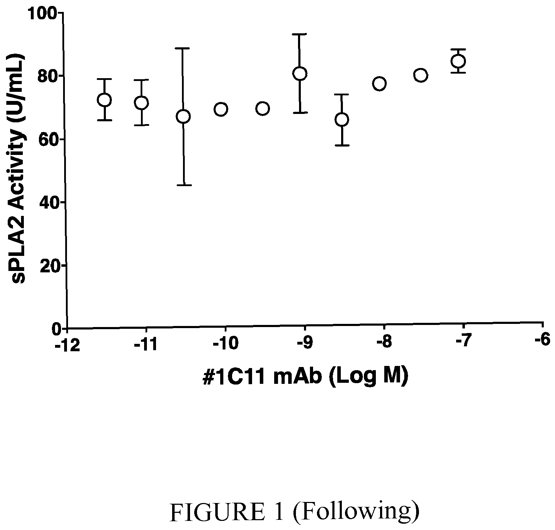

**Figure 1****:** Inhibition of sPLA2-GIB enzymatic activity by #14G9, #6F7 and #1C11 antibodies. Data were fitted with a 4PL logistic non-linear regression model and the relative IC50 values were estimated from the model.
**Figure 2****:** Dilution curves of anti-sPLA2-GIB antibodies in indirect ELISA. Data were fitted with a 4PL logistic non-linear regression model and the relative EC50 values were estimated from the model.
**Figure 3****:** Comparison of antibodies by competitive ELISA.
**Figure 4****:** *In vivo* inhibition by 1 mg of monoclonal antibody 14G9 of the inhibitory effect of 100 µg of sPLA2-GIB on the nuclear translocation of phosphoSTAT5 in CD4 T cells isolated from mice splenocytes. No significant effect was observed when injecting 900 µg of an isotype control mAb.
**Figure 5****:** Epitope identification: Non-covalent bonds in Fab#2B2/sPLA2-GIB complex, sPLA2-GIB backbone is in yellow, #2B2 Fab light chain is in light blue and #2B2 Fab heavy chain is in green.

**Table 1:** Description of 36 mAbs directed against pro-sPLA2-IB and sPLA2-IB. Antigens used for immunization. isotype. sPLA2 enzymatic activity inhibition and relative affinity for pro-sPLA2 and sPLA2-IB determined by indirect ELISA are listed for each subclones.
**Table 2:** ELISA IC50 towards recombinant human sPLA2-IB and recombinant human pro-sPLA2-IB determined by competitive ELISA.
**Table 3:** Determination of Kd towards recombinant human sPLA2-IB and recombinant human pro-sPLA2-IB by Surface Plasmon Resonance.
**Table 4:** Epitope mapping by competing ELISA. Results are expressed as percentage of signal after subtraction of the signal of the autologous competition with 0% representing maximum competition and 100% no competition.
**Table 5:** Summary of the analytical performances of sandwich ELISAs specific to pro-sPLA2-IB, sPLA2-IB or both forms.
**Table 6:** Distribution of sPLA2-IB, total sPLA2-IB and sPLA2-IB among apparently healthy Male and Female and correlation with age and BMI.
**Table 7:** Percentile distribution of pro-sPLA2-IB, sPLA2-IB and both forms among apparently healthy Male and Female.

### DEFINITIONS

As used herein, the term "PLA2-GIB" designates group IB pancreatic phospholipase A2. PLA2-GIB has been identified and cloned from various mammalian species. The human PLA2-GIB protein is disclosed, for instance, in Lambeau and Gelb (2008). The sequence is available on Genbank No. NP_000919.
The amino acid sequence of an exemplary human PLA2-GIB is shown below (SEQ ID NO: 1). 
MKLLVLAVLL TVAAADSGIS PRAVWQFRKM IKCVIPGSDP FLEYNNYGCY
CGLGGSGTPV DELDKCCQTH DNCYDQAKKL DSCKFLLDNP YTHTYSYSCS
GSAITCSSKN KECEAFICNC DRNAAICFSK APYNKAHKNL DTKKYCQS
 Amino acids 1 to 15 of SEQ ID NO: 1 (underlined) are a signal sequence, and amino acids 16 to 22 of SEQ ID NO: 1 (in bold) are a propeptide sequence.
Within the context of the invention, the term "PLA2-GIB" designates preferably human PLA2-GIB.

The human PLA2-GIB protein may be present under two distinct forms: an inactive pro form (pro-sPLA2-GIB), which is activated by proteolytic cleavage of a pro-peptide, leading to the mature secreted form (sPLA2-GIB). The term PLA2-GIB includes any form of the protein, such as the pro-form and/or the mature form. Typically, the mature secreted form comprises the sequence of amino acid residues 23-148 of SEQ ID NO: 1 (SEQ ID NO: 26) or any natural variants thereof, such as variants resulting from polymorphism or splicing. The term "pro-sPLA2-GIB" or "proPLA2-GIB" designates preferably a protein comprising the sequence of amino acid residues 16-148 of SEQ ID NO: 1 or any natural variants thereof, such as variants resulting from polymorphism or splicing.

Generally, the term "antibody" designates any immunoglobulin-type molecule comprising heavy and light chains. Typical antibodies possess the basic monomeric "H2L2" structure consisting of 2 Heavy and 2 Light chains. Each heavy chain is paired with a light chain. Heavy and light chains comprise a "variable region" or "variable domain". The variable domain of the heavy chain may be referred to as "VH." The variable domain of the light chain may be referred to as "VL." These domains are generally the most variable parts of an antibody and contain the antigen-binding sites. A light or heavy chain variable region (VL or VH) consists of a framework region interrupted by three hypervariable regions referred to as "complementarity determining regions" or "CDRs".

Antibody "fragments" designate any portion of an antibody that confers antigen binding. Fragments typically designate Fab, Fab'2, ScFv, or CDR.

Variants of an antibody include any chemically- or biologically-modified antibodies that retain antigen specificity. Particular variants are labelled antibodies, carrying a label (such as a marker, tracer, tag) allowing detection or capture of an antibody. Examples of such variants include fusion molecules formed by covalent or non-covalent association of the antibody with one or more proteins or peptides, such as a reporter peptide allowing the detection, or the quantification of the fusion molecule. In some embodiments, antibody variants of the invention can be engineered, such as biotinylated, to be detectable and/or quantifiable.

In preferred embodiments, antibodies and fragments and variants of the invention are isolated. An "isolated" antibody is one which has been separated from a component of its natural environment. In particular, the antibody may be purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g. Flatman et al., J. Chromatogr. B 848:79-87 (2007).

An antibody is "selective" for an antigen, or "selectively binds" an antigen when such antibody exhibits preferential binding to said antigen as compared to other molecules. Selectivity can be determined by competitive ELISA or Biacore assays. The difference in affinity/avidity that marks selectivity can be any detectable preference, such as e.g., a ratio of more than 1:1.1, or more than about 1:5, 1:10, 1:100, 1:1000 or more.

The term "competitively inhibits" indicates that the antibody can reduce or inhibit or displace the binding of a reference antibody to sPLA2-GIB. Competition assays can be performed using standard techniques such as, for instance, competitive ELISA or other binding assays. Typically, a competitive binding assay involves a purified target antigen, generally bound either to a solid substrate or cells, an unlabeled test antibody and a labeled reference antibody. Competitive inhibition is measured by determining the amount of labeled antibody bound in the presence of the test antibody. Usually the test antibody is present in excess, such as about 5 to 500 times the amount of reference antibody. Typically, for ELISA, the test antibody is in 100X excess, and for enzymatic methods, the test antibody in in 10X excess. When a test antibody present in excess inhibits or displaces at least 70% of the binding of the reference antibody to the antigen, it is considered as competitively inhibiting said reference antibody. In a specific embodiment, when a test antibody present in 100X excess inhibits or displaces at least 70%, more preferably at least 80% of the binding of the reference antibody to the antigen in ELISA, it is considered as competitively inhibiting said reference antibody. Preferred competing antibodies bind epitopes that share common amino acid residues.

### DETAILED DESCRIPTION OF THE INVENTION

The present invention relates to novel monoclonal antibodies that bind human PLA2-GIB protein, fragments of such antibodies, as well as their uses, particularly for detecting, quantifying or monitoring PLA2-GIB in a sample as well as for inhibiting sPLA2-GIB.

The inventors have previously identified sPLA2-GIB as playing a crucial role in the mechanism underlying the unresponsiveness of CD4 T lymphocytes, such as in viremic HIV-infected patients. The inventors have now generated anti-PLA2-GIB monoclonal antibodies using different types of immunogens. The immunogens were designed to generate antibodies with relevant specificities, in particular using a peptide containing essentially the pro region. The inventors produced and tested nearly 40 monoclonal antibodies and selected antibodies having most relevant specificity, affinity and activity. The sequences of selected antibodies were determined, as well as some epitopes. These antibodies, alone and in combinations, allow efficient detection and/or inhibition of PLA2-GIB. Also, they may be used to design methods for discriminating the pro- and secreted forms of PLA2-GIB in any sample.

In a first aspect, the present invention thus relates to monoclonal antibodies, or fragments thereof, that bind human PLA2-GIB. Particular antibodies of the invention bind selectively the mature secreted form of human PLA2-GIB. Other particular antibodies of the invention bind selectively the pro-form of human PLA2-GIB, or both the mature secreted form and the pro form of human PLA2-GIB. The monoclonal antibodies or fragments of the invention may be used to discriminate the forms of human PLA2-GIB. They may also be used to specifically inhibit the mature secreted form of human PLA2-GIB.

### Antibodies against sPLA2-GIB

In a particular aspect, the invention relates to monoclonal antibodies, or fragments thereof, that bind the mature secreted form of human PLA2-GIB (sPLA2-GIB), preferably that selectively bind sPLA2-GIB.

A particular example of such antibodies is 14G9. Antibody 14G9 was generated by immunization with sPLA2-GIB. 14G9 antibody was selected based on its very high affinity for sPLA2-GIB, its potent neutralizing activity, and its selectivity. The amino acid sequences of the heavy and light chains of 14G9 were determined, allowing recombinant production of this antibody. Furthermore, the epitope of 14G9 on sPLA2-GIB was also determined.

Upon testing, it was found that 14G9 is able to selectively bind sPLA2-GIB. It was also found that 14G9 can inhibit sPLA2-GIB in vitro and in vivo. This antibody thus exhibits remarkable properties for selectively detecting or inhibiting the protein. Further monoclonal antibodies prepared by the inventors were tested for their capacity to competitively inhibit binding of 14G9 to sPLA2-GIB. It was found that monoclonal antibodies 24B2, 22C6, 28A1, 28A3, 34G3, 22H5 and 7C5 can indeed competitively inhibit binding of 14G9 to sPLA2-GIB (see Table 4). Furthermore, it was verified that such competing antibodies can bind and neutralize PLA2-GIB (see Table 1), thus confirming the relevance of this group of antibodies.

In a particular embodiment, the invention thus relates to any monoclonal antibody, variants or fragments thereof, that bind the mature secreted form of human PLA2-GIB and competitively inhibit the binding of monoclonal antibody 14G9 to human sPLA2-GIB.

In a further particular embodiment, the invention relates to monoclonal antibodies, variants or fragments thereof, which bind an epitope comprising at least one amino acid residue selected from W3, R6, K7, K10, C77, Y75, G79 and S80 of human sPLA2-GIB protein (by reference to SEQ ID NO: 26). In a preferred aspect, antibodies, variants and fragments of the invention bind an epitope comprising at least 2, 3, 4, 5, 6, 7 or all of the amino acid residues selected from W3, R6, K7, K10, C77, Y75, G79 and S80 of human sPLA2-GIB protein. The epitope of monoclonal antibody 14G9 as determined by the inventors was found to involve amino acid residues W3, R6, and K7, for binding of the light chain, and amino acid residues K10, C77, Y75, G79 and S80, for binding of the heavy chain. The invention thus also relates to antibodies and fragments thereof which have in their epitopes at least one amino acid residue of the epitope of 14G9, preferably at least 2 or more.

In a more particular embodiment, a monoclonal antibody of the invention is antibody 14G9 or a variant or a fragment thereof.

The light and heavy chains variable region of antibody 14G9 are provided in SEQ ID NOs: 2 and 4, respectively.

In a more particular embodiment, the invention relates to a monoclonal antibody comprising (i) a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 2, and (ii) a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 4.
With reference to SEQ ID NO: 2, the three CDR regions correspond to the following amino acid residues:
- CDR-L1: amino acid residues QDVSTA,
- CDR-L2: amino acid residues WAS,
- CDR-L3: amino acid residues QQDYSTPPT.
With reference to SEQ ID NO: 4, the three CDR regions correspond to the following amino acid residues:
- CDR-H1: amino acid residues GYTFTNYW,
- CDR-H2: amino acid residues IDPSDTRT,
- CDR-H3: amino acid residues ARQTLYYEALDY.

In a more particular embodiment, the invention relates to a monoclonal antibody comprising the light chain variable region consisting, or consisting essentially, of SEQ ID NO: 2 and/or the heavy chain variable region consisting, or consisting essentially, of SEQ ID NO: 4.

In another particular embodiment, a monoclonal antibody of the invention is antibody 22C6, or a variant or a fragment thereof.

The amino acid sequences of 22C6 light chain variable region and heavy chain variable region are provided in SEQ ID NOs: 7 and 9, respectively.

In a more particular embodiment, the invention relates to a monoclonal antibody comprising (i) a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 7, and (ii) a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 9.
With reference to SEQ ID NO: 7, the three CDR regions correspond to the following amino acid residues:
- CDR-L1: amino acid residues SSISPNF,
- CDR-L2: amino acid residues RTS,
- CDR-L3: amino acid residues HQGNSLPLT.
With reference to SEQ ID NO: 9, the three CDR regions correspond to the following amino acid residues:
- CDR-H1: amino acid residues GHSITDDNYY,
- CDR-H2: amino acid residues ITSAGNT,
- CDR-H3: amino acid residues ARDSTDGDFYFEK.

In a more particular embodiment, the invention relates to a monoclonal antibody comprising the light chain variable region consisting, or consisting essentially, of SEQ ID NO: 7 and/or the heavy chain variable region consisting, or consisting essentially, of SEQ ID NO: 9.

In another more particular embodiment, a monoclonal antibody of the invention is antibody 24B2, or a variant or a fragment thereof.

The amino acid sequences of 24B2 light chain variable region and heavy chain variable region are provided below in SEQ ID NOs: 11 and 13, respectively.

In a more particular embodiment, the invention relates to a monoclonal antibody comprising (i) a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 11, and (ii) a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 13.
With reference to SEQ ID NO: 11, the three CDR regions correspond to the following amino acid residues:
- CDR-L1: amino acid residues ESVDNYGISF,
- CDR-L2: amino acid residues RAS,
- CDR-L3: amino acid residues QQSSRDLFT.
With reference to SEQ ID NO: 13, the three CDR regions correspond to the following amino acid residues:
- CDR-H1: amino acid residues GHSITDDNYY,
- CDR-H2: amino acid residues ITSSGST,
- CDR-H3: amino acid residues ARDSTDGDFYFEK.

In a more particular embodiment, the invention relates to a monoclonal antibody comprising the light chain variable region consisting, or consisting essentially, of SEQ ID NO: 11 and/or the heavy chain variable region consisting, or consisting essentially, of SEQ ID NO: 13.

The antibodies against sPLA2-GIB may be selective and bind essentially only the mature secreted form of the protein. In particular, 14G9 binds sPLA2-GIB and essentially does not bind the pro form of the protein.

### Antibodies against pro-PLA2-GIB

In a particular aspect, the invention relates to monoclonal antibodies, or fragments thereof, that bind the pro- form of human PLA2-GIB (proPLA2-GIB), preferably that selectively bind proPLA2-GIB.

A particular example of such antibodies is 8G11. Antibody 8G11 was generated by immunization with a peptide comprising a N-terminal portion of PLA2-GIB. The antibody was selected based on its very high affinity for proPLA2-GIB and its selectivity for the prop form. The amino acid sequences of the heavy and light chains of 8G11 were determined, allowing recombinant production of this antibody. Further monoclonal antibodies prepared by the inventors were tested for their capacity to competitively inhibit binding of 8G11 to proPLA2-GIB. It was found that monoclonal antibody 18D9 can indeed competitively inhibit binding of 8G11 to proPLA2-GIB (see Table 4).

In a particular embodiment, the invention thus relates to any monoclonal antibody, or variants or fragments thereof, that bind the pro form of human PLA2-GIB and competitively inhibit the binding of monoclonal antibody 8G11 to human sPLA2-GIB.

In a more particular embodiment, a monoclonal antibody of the invention is antibody 8G11, or a variant or a fragment thereof.

The amino acid sequences of 8G11 light chain variable region and heavy chain variable region are provided in SEQ ID NOs: 15 and 17, respectively.

In a more particular embodiment, the invention relates to a monoclonal antibody comprising (i) a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 15, and (ii) a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 17.
With reference to SEQ ID NO: 15, the three CDR regions correspond to the following amino acid residues:
- CDR-L1: amino acid residues KSVSTSGYSY,
- CDR-L2: amino acid residues LVS.
With reference to SEQ ID NO: 17, the three CDR regions correspond to the following amino acid residues:
- CDR-H1: amino acid residues GYTFNDDE,
- CDR-H2: amino acid residues IDPETGGT,
- CDR-H3: amino acid residues QLIFHGNPYYCMDY.

In a more particular embodiment, the invention relates to a monoclonal antibody comprising the light chain variable region consisting, or consisting essentially, of SEQ ID NO: 15 and/or the heavy chain variable region consisting, or consisting essentially, of SEQ ID NO: 15.

The antibodies against proPLA2-GIB may be selective and bind essentially only the pro form of the protein. In particular, 8G11 binds proPLA2-GIB and essentially does not bind the sPLA2-GIB form of the protein.

### Dual antibodies against the sPLA2-GIB and pro-PLA2-GIB

In a particular aspect, the invention relates to monoclonal antibodies, or fragments thereof, that bind both the mature secreted form (sPLA2-GIB) and the pro- form (pro-sPLA2-GIB) of human PLA2-GIB protein.

A particular example of such antibodies is 1C11. Antibody 1C11 was generated by immunization with sPLA2-GIB. The antibody was selected based on its very high affinity for both proPLA2-GIB and sPLA2-GIB. The amino acid sequences of the heavy and light chains of 1C11 were determined, allowing recombinant production of this antibody. Further monoclonal antibodies prepared by the inventors were tested for their capacity to competitively inhibit binding of 1C11 to proPLA2-GIB and/or sPLA2-GIB. It was found that monoclonal antibodies 4G1 1, 7H12 and 7E2 can indeed competitively inhibit binding of 1C11 (see Table 4).

In a particular embodiment, the invention thus relates to any monoclonal antibody, or variants or fragments thereof, that binds human PLA2-GIB and competitively inhibits the binding of monoclonal antibody 1C11 to human pro-PLA2-GIB and/or to human sPLA2-GIB, preferably to human pro-PLA2-GIB and to human sPLA2-GIB.

In a more particular embodiment, a monoclonal antibody of the invention is antibody 1C11, or a variant or a fragment thereof.

The amino acid sequences of 1C11 light chain variable region and heavy chain variable region are provided in SEQ ID NOs: 19 and 21, respectively.

In a more particular embodiment, the invention relates to a monoclonal antibody comprising (i) a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 19, and (ii) a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 21.
With reference to SEQ ID NO: 19, the three CDR regions correspond to the following amino acid residues:
- CDR-L1: amino acid residues SSVSY,
- CDR-L2: amino acid residues EIS,
- CDR-L3: amino acid residues QSWNFOLLS.
With reference to SEQ ID NO: 21, the three CDR regions correspond to the following amino acid residues:
- CDR-H1: amino acid residues GYAFSSFW,
- CDR-H2: amino acid residues IYPGDGDI,
- CDR-H3: amino acid residues VRGWAWFPY.

In a more particular embodiment, the invention relates to a monoclonal antibody comprising the light chain variable region consisting, or consisting essentially, of SEQ ID NO: 19 and/or the heavy chain variable region consisting, or consisting essentially, of SEQ ID NO: 21.

Another particular example of such antibodies is 7A10. Antibody 7A10 was generated by immunization with sPLA2-GIB. The antibody was selected based on its very high affinity for both proPLA2-GIB and sPLA2-GIB. The amino acid sequences of the heavy and light chains of 7A10 were determined, allowing recombinant production of this antibody. Further monoclonal antibodies prepared by the inventors were tested for their capacity to competitively inhibit binding of 7A10 to proPLA2-GIB and/or sPLA2-GIB. It was found that monoclonal antibodies 36G10, 3B2, 18A6 and 13H8 can indeed competitively inhibit binding of 7A10 (see Table 4).

In a particular embodiment, the invention thus relates to any monoclonal antibody, or fragments thereof, that binds human PLA2-GIB and competitively inhibits the binding of monoclonal antibody 7A10 to human pro-PLA2-GIB and/or to human sPLA2-GIB, preferably to human pro-PLA2-GIB and to human sPLA2-GIB.

In a more particular embodiment, a monoclonal antibody of the invention is antibody 7A10, or a variant or a fragment thereof.

The amino acid sequences of 7A10 light chain variable region and heavy chain variable region are provided in SEQ ID NOs: 23 and 25, respectively.

In a more particular embodiment, the invention relates to a monoclonal antibody comprising (i) a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 23, and (ii) a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 25.
With reference to SEQ ID NO: 23, the three CDR regions correspond to the following amino acid residues:
- CDR-L1: amino acid residues QGISIW,
- CDR-L2: amino acid residues KAS,
- CDR-L3: amino acid residues LQSQSYPLT.
With reference to SEQ ID NO: 25, the three CDR regions correspond to the following amino acid residues:
- CDR-H1: amino acid residues GFSLTSYG,
- CDR-H2: amino acid residues IWGDGST,
- CDR-H3: amino acid residues AKEGGLRRGVYFDY.

In a more particular embodiment, the invention relates to a monoclonal antibody comprising the light chain variable region consisting, or consisting essentially, of SEQ ID NO: 23 and/or the heavy chain variable region consisting, or consisting essentially, of SEQ ID NO: 25.

The antibodies of the invention are typically Immunoglobulin G (IgG) antibodies. IgG antibody can be divided into 4 isotypes: IgG1, IgG2, IgG3 and IgG4, depending on differences in the amino sequence of the constant region of the heavy chain. Antibodies of the invention can belong to any IgG isotype.

### Nucleic acids, vectors and host cells

The invention also relates to nucleic acids encoding an antibody or fragment as defined above, as well as cloning or expression vectors containing such nucleic acids, and recombinant host cells.

In another aspect, the present invention concerns nucleic acid molecules encoding an antibody of the invention, or a nucleic acid complementary to said encoding sequence. Preferably, the nucleic acid is an isolated or purified nucleic acid.

The nucleic acid can be DNA (cDNA or gDNA), RNA, or a mixture thereof. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis. The nucleic acid according to the invention may be deduced from the sequence of the antibody according to the invention and codon usage may be adapted according to the host cell in which the nucleic acid shall be transcribed. These steps may be carried out according to methods well known to one of skill in the art and some of which are described in the reference manual Sambrook *et al.* (Sambrook J, Russell D (2001) Molecular cloning: a laboratory manual, Third Edition Cold Spring Harbor).

The nucleic acid of the invention may encode an amino acid sequence comprising the light chain and/or an amino acid sequence comprising the heavy chain of the antibody, or may be complementary to such encoding sequence.

Specific examples of such nucleic acid sequences include the sequences comprising anyone of SEQ ID NOs: 3, 5, 6, 8, 10, 12, 14, 16, 18, 20, 22 and 24, and the complementary sequence thereto.

The present invention further provides a vector comprising a nucleic acid of the invention. Optionally, the vector may comprise several nucleic acids of the invention. In particular, the vector may comprise a nucleic acid of the invention operably linked to a regulatory region, i.e. a region comprising one or more control sequences. Optionally, the vector may comprise several nucleic acids of the invention operably linked to several regulatory regions.

The term "control sequences" means nucleic acid sequences necessary for expression of a coding region. Control sequences may be endogenous or heterologous. Well-known control sequences and currently used by the person skilled in the art will be preferred.

Such control sequences include, but are not limited to, promoter, signal peptide sequence and transcription terminator.

The term "operably linked" means a configuration in which a control sequence is placed at an appropriate position relative to a coding sequence, in such a way that the control sequence directs expression of the coding region.

The present invention further relates to the use of a nucleic acid or vector according to the invention to transform, transfect or transduce a host cell.

The present invention also provides a host cell comprising one or several nucleic acids of the invention and/or one or several vectors of the invention.

The term "host cell" also encompasses any progeny of a parent host cell that is not identical to the parent host cell due to mutations that occur during replication.

Suitable host cells for cloning or expression of antibody-encoding vectors include prokaryotic or eukaryotic cells described herein.

For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell lysate in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22: 1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See, e.g., US Patent Nos. 5,959,177, 6,040,498, 6,420,548, 7,125,978, and 6,417,429.

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse Sertoli cells (TM4 cells as described, e.g., in Mather, Biol. Reprod. 23:243-251 (1980)); monkey kidney cells (CV1); African green monkey kidney cells (VERO- 76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N. Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255- 268 (2003).

The present invention also concerns a method for producing an antibody of the invention, comprising culturing a host cell comprising a nucleic acid of the invention or a vector of the invention, as provided above, under conditions suitable for expression of the antibody, and optionally recovering the antibody from the host cell or from the host cell culture medium. Optionally, the recovered antibody may be further purified or isolated. Suitable media, culture conditions and production method are well-known by the skilled person and can be easily chosen according to the host cell and the antibody to be produced.

### Methods of detection, dosage, diagnosis

In a further aspect, the present invention relates to a method for detecting human PLA2-GIB using an antibody or fragment as defined above. The method can be used or implemented for detecting the presence of the mature secreted form (sPLA2-GIB), the pro- form (pro-sPLA2-GIB) and/or both forms of the human PLA2-GIB protein. Such method or antibodies may be used to detect or dose (or quantify) the presence or amount of PLA2-GIB in a sample, as well as to assess the ratio of pro and mature secreted forms thereof. The methods are particularly useful for detecting or monitoring pathological conditions in subjects that are associated with an activity of PLA2-GIB.

In a particular embodiment, the method comprises a) providing a sample, and b) detecting the presence or amount of the mature secreted form (sPLA2-GIB), the pro- form (pro-PLA2-GIB) and/or both forms of the human PLA2-GIB protein in the sample using an antibody or fragment as defined above.

In a particular embodiment, the antibody is selected from 14G9, 24B2, 22C6, 28A1, 28A3, 34G3, 22H5 and 7C5, or fragments or variants thereof, and the method allows detecting the presence or amount of sPLA2-GIB.

In another particular embodiment, the antibody is selected from 8G11 and 18D9, or fragments or variants thereof, and the method allows detecting the presence or amount of proPLA2-GIB.

In another particular embodiment, the antibody is selected from 1C11, 7A10, 36G10, 3B2, 18A6, 13H8 4G11, 7H12 and 7E2, or fragments or variants thereof, and the method allows detecting the total amount of PLA2-GIB.

Typically, the detecting step b) comprises the steps of (i) contacting the sample, or biological replicates of the sample, with anyone of the monoclonal antibodies or fragments or variants thereof herein described, and (ii) detecting the presence of an antibody : antigen complex formed in step (i).

Detecting the presence of an antibody: antigen complex can be performed by any technique well known per se by the skilled person and extensively described in the literature. Typically, detection can be performed with a second antibody against PLA2-GIB, preferably that binds an epitope distinct from that of the first capture antibody. Alternatively, the capture antibody may be labelled and the detection step may comprise measuring the amount of label in the reaction, typically after removing unbound antibodies.

In a particular embodiment, the sample is divided in replicates to detect separately the mature secreted form (sPLA2-GIB), the pro- form (pro-PLA2-GIB) and/or both forms of the human PLA2-GIB protein using any combination of antibodies, variants or fragments of the invention.
in a more particular embodiment, the sample is divided in at least two replicates, one replicate is contacted with an antibody selected from 14G9, 24B2, 22C6, 28A1, 28A3, 34G3, 22H5 and 7C5, or fragments or variants thereof, allowing detection of the presence or amount of sPLA2-GIB; and one is contacted with an antibody selected from 8G11 and 18D9, or fragments or variants thereof, allowing detection of the presence or amount of proPLA2-GIB.

In a more particular embodiment, the sample is divided in a further replicate which is contacted with an antibody selected from 1C11, 7A10, 36G10, 3B2, 18A6, 13H8 4G11, 7H12 and 7E2, or fragments or variants thereof, allowing detection of the total amount of PLA2-GIB.

In a preferred embodiment, the method utilizes 14G9 or a variant or fragment thereof for detection of the presence or amount of sPLA2-GIB, 8G11 or a variant or fragment thereof for detection of the presence or amount of proPLA2-GIB, and/or 7A10 or a variant or fragment thereof for detection of the total amount of PLA2-GIB in the sample.

The antibody may be immobilized on a surface or particle, or not. The reaction can be performed in any appropriate support or container, such as microplate, on a chip, in a bottle, etc.

Preferably, the detecting step (ii) utilizes a labelled antibody, such as an conjugated to a reporting group or engineered to be detectable, more preferentially said antibody is biotinylated. In a more particular embodiment, the antibody used for detecting the complex is selected from the monoclonal antibodies 1C11, 4G11, 7H12 and 7E2, and variants and fragments thereof, preferentially said antibody is 1C11 or a variant or fragment thereof, even more preferentially said antibody is biotinylated antibody 1C11.

The method may be used with any sample such as any biological sample, such as a serum sample, a plasma sample, whole blood, biological fluids, etc. In more particular embodiments, the sample used in any of the methods above described is obtained from a subject. The sample may be treated prior to detection, such as by dilution, concentration, enrichment, etc. It may be lyophilized, sterilized, frozen, etc., before use in a method of the invention.

In particular embodiments, the method is used to assess the ratio of pro and mature secreted forms of PLA2-GIB.

In other particular embodiments, the methods above described may be used for monitoring pathological conditions in subjects that are associated with an activity of PLA2-GIB. Example of pathological condition associated with an activity of PLA2-GIB is the unresponsiveness of CD4 T lymphocytes, such as in viremic HIV-infected patient.

In a further aspect, the present invention relates to kits comprising a least one antibody, or fragment thereof, as herein described and a reagent to perform, detect or quantify an immune reaction, or an antibody-antigen complex.

Further aspects and advantages of the invention will be disclosed in the following experimental section.

### EXAMPLES

### MATERIALS AND METHODS

### 1 -Production of human recombinant pro-sPLA2-GIB and sPLA2-GIB proteins

Human pro-sPLA2-GIB and sPLA2-GIB were produced according to published procedures (Singer et al. 2002; Rouault et al. 2007).

### 2- Generation of anti-sPLA2-GIB and anti-pro-sPLA2-GIB antibodies

A first series of immunization was performed by immunizing mice with recombinant human sPLA2-GIB and recombinant human pro-sPLA2-GIB. A second series of immunization was performed by immunizing mice with a 18 amino-acids peptide (sequence NH2-DSGISPRAVWQFRKMIKC-COOH) corresponding to the human PLA2-GIB pro-peptide followed by the first 11 amino-acids of the mature active sPLA2-GIB (see amino acid residues 16-33 of SEQ ID NO: 1). mAb were purified by protein A affinity and quantified. The Ig class of mAbs was determined with a commercial mouse mAb isotyping kit according to the manufacturer's instructions.

### 3- Inhibition of sPLA2-GIB enzymatic activity

Inhibition of sPLA2-GIB enzymatic activity by the different monoclonal antibody (mAb) was tested according to published procedures (Rouault et al. 2007). Briefly, recombinant human sPLA2-GIB (0.1 nM) was incubated with increasing volumes of hybridomas cell culture supernatants (data not shown) or increasing amount (0.1, 0.3, 1, and 3 µg) of a purified rabbit polyclonal immune-serum targeted towards human sPLA2-GIB or of purified monoclonal antibodies for 30 min at 37°C. sPLA2-GIB enzymatic activity was then measured using [3H]-oleate-radiolabeled autoclaved E. coli membranes as phospholipid substrate in sPLA2-GIB activity buffer. Reactions were stopped by addition of 300 µL of stop buffer, mixtures were then centrifuged at 10000g for 5 min, and the supernatants containing released [3H]-oleate were counted.

Results were confirmed using a commercial fluorimetric enzymatic activity assay. Recombinant human sPLA2-GIB (3 nM) was incubated with increasing amount of purified monoclonal antibodies for 120 min at room temperature. sPLA2-GIB enzymatic activity was then measured using a selective fluorimetric method (AteroDX® sPLA2 Activity. Aterovax. Paris. France). Results are expressed in Unit per mL of sample (U/mL), with one unit defined as the amount of sPLA2-GIB enzyme which catalyzes the release of one nmol of product in one min. Limit of detection of the assay is 17 U/mL with upper linear analytical range of 232 U/mL and functional sensitivity (20%) is 21 U/mL. Average within-run variability and average intra-assay variability are 5.9% and 8.9%. respectively. Results of both methods were analyzed with a 4-parameter fit analysis using Prism™ software (Graph Pad) and reported as IC50.

### 4- Indirect ELISA

Microplate wells were typically coated with 100 ng of recombinant human sPLA2-GIB or pro-sPLA2-GIB in PBS pH 7.5, overnight at 4°C. Sample wells were washed three times with PBS containing 0.05% Tween 20. After final washing, sample wells were treated with blocking solution containing 1% bovine serum albumin (BSA) in PBS buffer for 60 min at room temperature. Following washing with PBS containing 0.05% Tween 20, increasing amounts (10 ng/mL up to 3 µg/mL) of mAb directed against human pro-sPLA2-GIB or sPLA2-GIB were added to antigen-coated wells. and incubated for 120 min at room temperature. Following washing with PBS containing 0.05% Tween 20, the binding of mAb was detected with an HRP-conjugated goat anti-mouse IgG or IgA polyclonal antibody reagent and developed using colorimetric substrate. The dependency of the signals on the concentrations of antigen in solution was analyzed with a 4-parameter fit analysis using Prism™ software (Graph Pad) and reported as EC50.

### 5- Competitive ELISA

Binding affinities of the antibodies to pro-sPLA2-GIB and sPLA2-GIB were measured by plate-based competition immunoassay. For the competition ELISA, briefly, constant amounts of antibody at different levels were premixed with serial dilutions of antigen protein, human sPLA2-GIB or pro-sPLA2-GIB, ranging from 0 to 10 µg/ml, and incubated for two hours at room temperature to reach pseudo-binding equilibrium between the antibody and antigen. These solutions were then transferred to 96-well sPLA2-GIB or pro-sPLA2-GIB pre-coated plates to allow the free-antibody in the mixtures to bind to plate-coated sPLA2-GIB or pro-sPLA2-GIB. The plates were typically coated with 30 to 100 ng of human sPLA2-GIB or pro-sPLA2-GIB protein in PBS solution overnight at 4° C followed by BSA nonspecific blocking. After washing off excess antibody in solution, plate-bound antibodies were detected with an HRP-conjugated goat anti-mouse IgG or IgA polyclonal antibody reagent and developed using colorimetric substrate. The dependency of the signals on the concentrations of antigen in solution was analyzed with a 4-parameter fit analysis using Prism™ software (Graph Pad) and reported as IC50.

### 6- Affinity determination by SPR

The affinity constant (KD) of the pro-sPLA2-GIB and sPLA2-GIB binding to the selected antibodies described above were determined by surface kinetics on a real-time biosensor surface plasmon resonance assay (BIAcore™). More specifically, the affinity of the antibodies for human pro-sPLA2-GIB and sPLA2-GIB was measured using a BIAcore® 2000. The antibody was captured on an anti-mouse IgG surface and exposed to various concentrations of recombinant pro-sPLA2-GIB and sPLA2-GIB protein. Kinetic analysis using BIAevaluation™ software was performed to obtain the association and dissociation rate constants.

### 7- Epitope mapping and mAb competition

Microplate wells were typically coated with 10 ng of recombinant human sPLA2-GIB or pro-sPLA2-GIB in PBS pH 7.5, overnight at 4°C. Sample wells were washed three times with PBS containing 0.05% Tween 20. After final washing, sample wells were treated with blocking solution containing 1% bovine serum albumin (BSA) in PBS buffer for 60 min at room temperature. Following washing with PBS containing 0.05% Tween 20, 10 ng of biotinylated mAb (#14G9, #1C11, #8G11 or #7A10) were added in the presence of 1 µg of non-biotinylated mAb to antigen-coated wells and incubated for 120 min at room temperature. Following washing with PBS containing 0.05% Tween 20, the binding of mAb was detected with an strepatavidin-HRP conjugate and developed using colorimetric substrate.

### 8- Sequencing of mAb variable regions

Variable regions of the mAbs were sequenced according to classical procedures. Briefly, highly pure RNA was extracted from hybridoma cells and complementary DNA strand were synthesized using high-fidelity reverse-transcriptase. PCR products generated using high-fidelity PCR and degenerate primers targeting specifically cDNA encoding for antibody heavy chain and light chain were directly sequenced (double strand sequencing). cDNA sequences were analyzed and assembled. Peptidic sequences were translated and validated according to antibody structure.

### 9- Development of sPLA2-GIB and pro-sPLA2-GIB sandwich ELISAs

Sandwich ELISAs specific to pro-sPLA2-GIB, sPLA2-GIB or both forms were constructed by using the mAb described above. For the detection of pro-sPLA2-GIB, the #8G11 mAb was used as a capture antibody and the #1C11 mAb was used as a revelation mAb. For the detection of sPLA2-GIB, the #14G9 mAb was used as a capture antibody and the #1C11 mAb was used as a revelation mAb. For the detection of both forms, the #7A10 mAb was used as a capture antibody and the #1C11 mAb was used as a revelation mAb. Different parameters such as nature of microplate, final volume in the well, time and temperature of incubation, nature and concentrations of streptavidin-HRP, composition of assay buffer, nature and concentration of added detergents, were studied to optimize the assays. Typical assay conditions were as follows: #8G11- or #14G9-coated microplate was incubated for one hour with recombinant human pro-sPLA2-GIB or sPLA2-GIB standards (varying concentrations of protein in assay buffer) to generate a calibration curve. EDTA plasma samples were diluted (5- to 50-fold) in dilution buffer to their respective wells and the ELISA plate was incubated for 1 h at room temperature. After aspiration, the wells were washed 3 times with PBS containing Tween 20 0.05%, and the biotinylated #1C11 conjugate was added to the wells for 30 min at room temperature. Following washing with PBS containing 0.05% Tween 20, the binding of mAb was detected by treatment with a Streptavidin-HRP conjugate for 15 min at room temperature. TMB was added, reaction was stopped and absorbance at 450 nm was determined on a microplate reader. Signals were analyzed with a 4-parameter fit analysis using Prism™ software (Graph Pad).

### 10- Determination of sPLA2-GIB and pro-sPLA2-GIB concentrations in human plasma samples

EDTA plasma samples from a commercial cohort of 99 apparently healthy donors with known age, gender and Body Mass Index (Bioreclamation IVT) were tested with the three different ELISA assays detecting pro-sPLA2-GIB, sPLA2-GIB and both forms (total PLA2-GIB), according to the conditions developed above. Correlations between parameters were evaluated using non-parametric tests (Spearman R).

### RESULTS

### Generation of anti-sPLA2-GIB and anti-pro-sPLA2-GIB antibodies

Monoclonal antibodies were produced by immunizing mice with recombinant human pro-sPLA2-GIB, recombinant human sPLA2-GIB or a 18 amino-acids peptide corresponding to the human sPLA2-GIB pro-peptide followed by the first 11 amino-acids of the mature active sPLA2-GIB. The antigens used to generate each subclone are described in Table 1.

### Screening of antibodies by inhibition of sPLA2 enzymatic activity

The ability of the different mAbs to inhibit sPLA2-GIB enzymatic activity was tested by incubating radiolabeled membranes with recombinant human sPLA2-GIB in the presence of increasing amounts of purified monoclonal antibodies. Results are expressed as percentage of inhibition of enzymatic activity observed with the highest concentration of mAb tested and are presented in Table 1.

### Comparison of antibodies by indirect ELISA

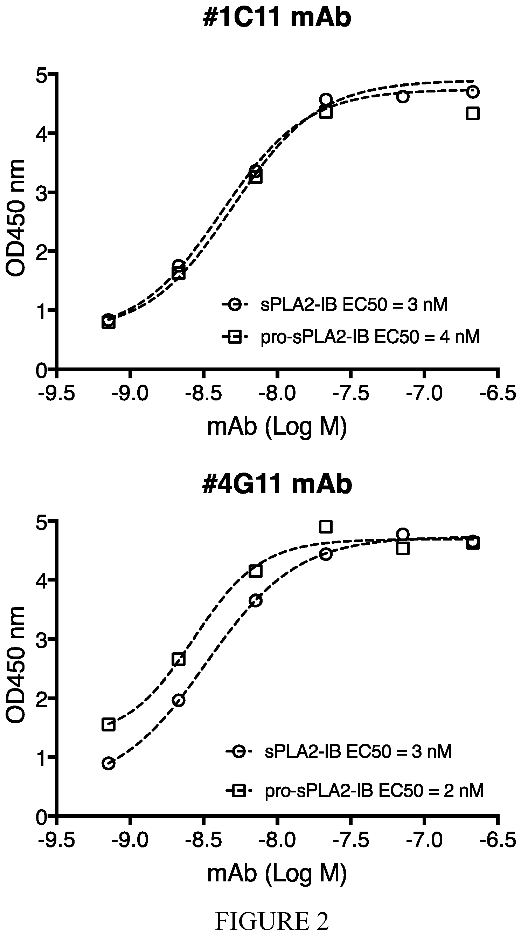

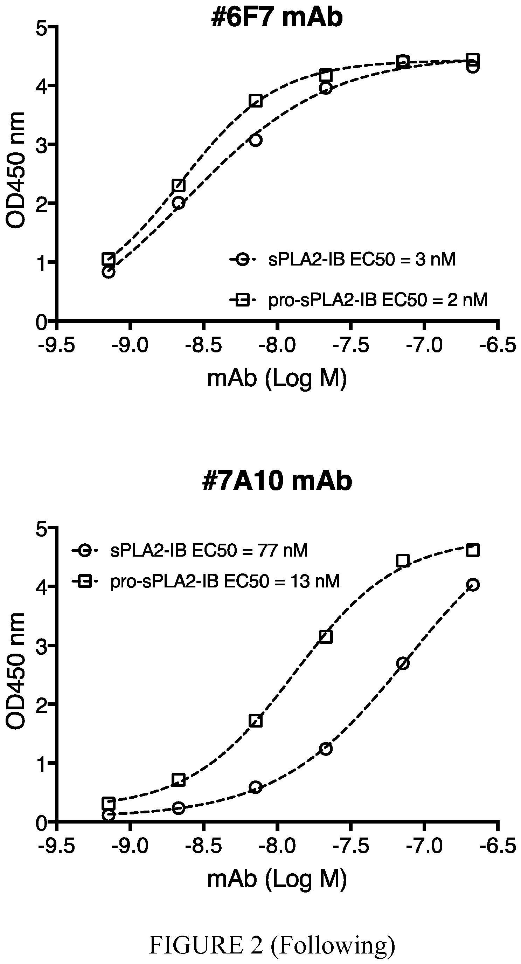

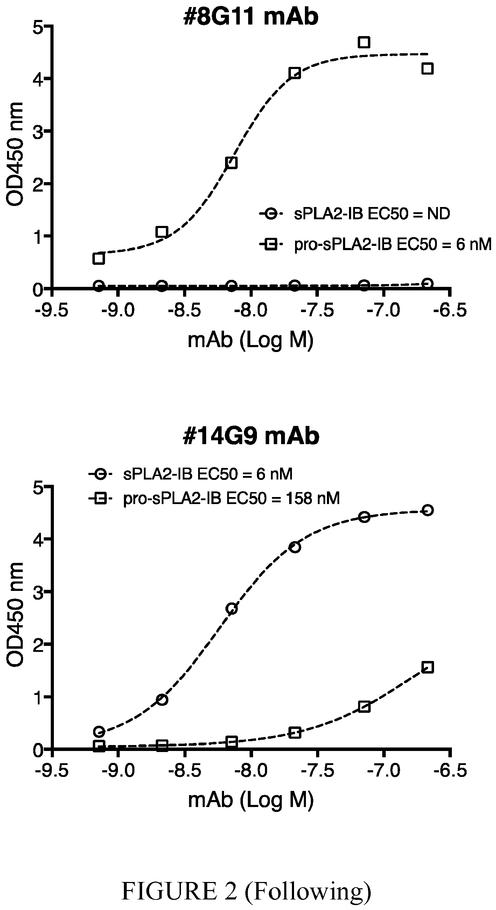

The different mAbs were compared in an indirect ELISA procedure by coating microplates with human sPLA2-GIB or pro-sPLA2-GIB and detecting bound mAbs by an HRP-conjugated goat anti-mouse IgG polyclonal antibody reagent. Example of typical signal curves are presented in Figure 2 and EC50 estimated from those profiles are presented in Table 1.

### Affinity determination of antibodies by competitive ELISA

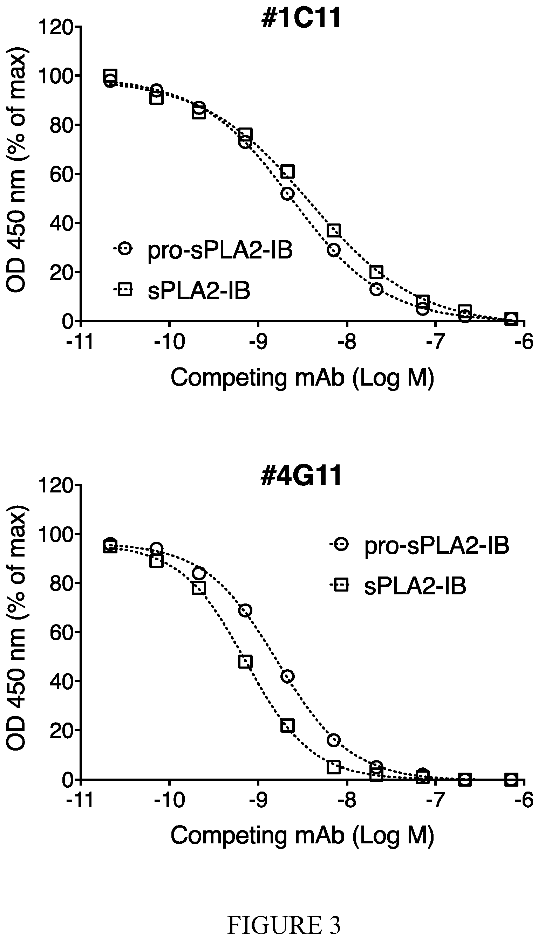

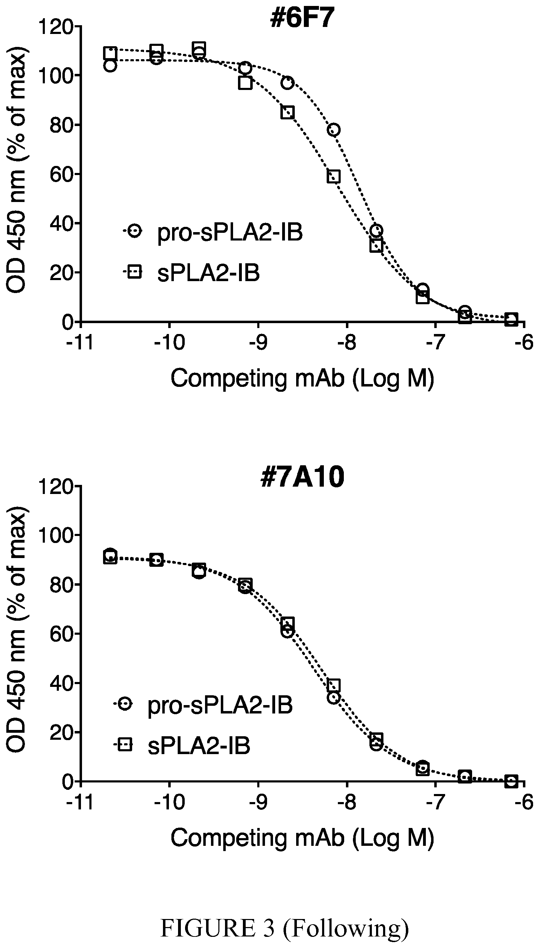

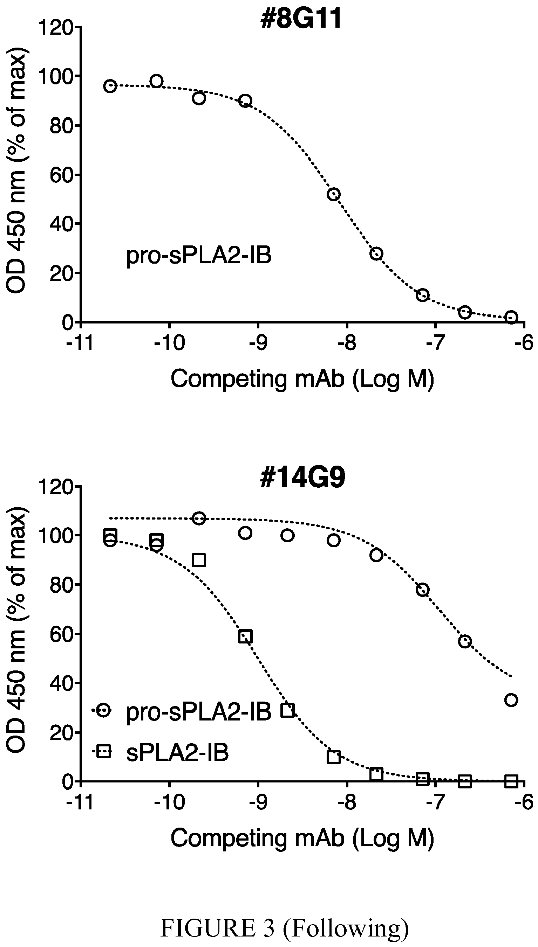

The respective binding affinities of the antibodies to human pro-sPLA2-GIB and human sPLA2-GIB were measured by plate-based competition immunoassay. Briefly, constant amounts of antibody at different levels were premixed with serial dilutions of antigen proteins to reach pseudo-binding equilibrium between the antibody and antigen. These solutions were then transferred to antigen pre-coated plates to allow the free-antibody in the mixtures to bind to plate-coated sPLA2-GIB or pro-sPLA2-GIB. Plate-bound antibodies were detected with an HRP-conjugated polyclonal antibody. Examples of the titration curves are presented in Figure 3. Data were fitted with a 4PL logistic non-linear regression model and the relative IC50 values were estimated from the model (Table 2).

### Affinity determination of antibodies by SPR

The affinity constant (KD) of the pro-sPLA2-GIB and sPLA2-GIB binding to the selected antibodies described above were determined by surface kinetics on a real-time biosensor surface plasmon resonance assay (BIAcore™). More specifically, the affinity of the antibodies for human pro-sPLA2-GIB and sPLA2-GIB was measured using a BIAcore® 2000. The antibody was captured on an anti-mouse IgG surface and exposed to various concentrations of recombinant pro-sPLA2-GIB and sPLA2-GIB protein. Kinetic analysis using BIAevaluation™ software was performed to obtain the association and dissociation rate constants (Table 3).

### Epitope mapping and mAb Competition

Epitope mapping of the #14G9, #1C11, #8G11 and #7A10 mAbs was evaluated by competition ELISA. Biotinylated mAbs #14G9, #1C11, #8G11 or #7A10 were added to sPLA2-GIB or pro-sPLA2-GIB coated plates in the presence of an excess of un-biotinylated mAb. Bound mAbs were then detected with Streptavidin-HRP conjugate. Results were expressed as percentage of signal, after subtraction of the autologous competition, with 0% representing maximum competition and 100% no competition (Table 4).

### Sequencing of variable regions

The amino acid and nucleic acid sequences of the variable regions of light and heavy chains of antibodies of the invention were determined and are presented in the sequence listing.

### Detection of pro-sPLA2-GIB, sPLA2-GIB or both forms by sandwich ELISAs

Sandwich ELISAs specific to pro-sPLA2-GIB, sPLA2-GIB or both forms were developed using adapted mAb pairs (see Table 5). The #8G11 mAb was used to specifically capture the enzyme in its zymogen inactive form. The #14G9 subclone, as a highly specific mAb for the mature sPLA2-GIB, was used to capture the mature enzyme.

The #7A10 mAb was used to capture both forms. Captured antigens were detected using a biotinylated conjugate of the highly affine #1C11 subclone. The different assay conditions were optimized and analytical performances of three different sandwich ELISAs, specific to pro-sPLA2-GIB, sPLA2-GIB and both forms, respectively, were evaluated. A summary of the analytical performances of these three different assays is presented in Table 5.

Assay accuracies were tested by spiking two human serum samples with pro-sPLA2-GIB or sPLA2-GIB. Percentages of recovery were between 80% and 120% for the three assays, demonstrating that matrix has limited effect. Assay limit of detections were estimated based on a mean + 3SD evaluation from the zero calibrator and determined to be 0.1 ng/mL, 0.02 ng/mL and 0.10 ng/ml for the pro-sPLA2-GIB, sPLA2-GIB or both forms assays, respectively. As human serum samples contained endogenous amount of pro-sPLA2-GIB and sPLA2-GIB, human serum samples were first depleted with #8G11 mAb or #14G9 mAb, before testing them for lower limit of quantitation. LLOQ was estimated based on the mean optical density of depleted human serums and to be 0.04 ng/mL, 0.02 ng/mL and 0.08 ng/mL, for the pro-sPLA2-GIB, sPLA2-GIB or both forms assays, respectively. Based on evaluation of samples spiked with increasing amount of recombinant pro-sPLA2-GIB or sPLA2-GIB, assay HLOQ was determined to be 5 ng/mL, 1 ng/mL or 5 ng/mL, for the pro-sPLA2-GIB, sPLA2-GIB or both forms assays, respectively. Intra-run and inter-run CVs (assay precision) were determined by repeatedly testing 3 human serums distributed across the dynamic range. Intra-run and inter-runs CVs were found to range between 2.3% and 14.5% (see table 5 for details). No significant interferences were observed when testing six human samples with high Rheumatoid Factor levels (up to 600 UI/mL) for the three different assays. Assay linearity was determined by testing serially diluted high levels samples. Mean % of recovery were between 80% and 120%, demonstrating good linearity for the three assays.

### Determination of the concentrations of pro-sPLA2-GIB, sPLA2-GIB or both forms in human plasma samples

The ELISA sandwich methods described above were used to examine the pro-sPLA2-GIB and sPLA2-GIB distribution by age, gender, and Body Mass Index (BMI) in a total of 99 samples from 50 apparently healthy males and 49 apparently healthy females in the clinically relevant age range of 25-59 years. Mean (SD) age of this population was 41.5 (9.8) years old and mean (SD) BMI was 30.5 (7.9).

In the total analyzed population, pro-sPLA2-GIB levels ranged from 1.0 to 16.3 ng/mL. The mean (SD) pro-sPLA2-GIB level was 7.8 (2.8) ng/mL and the median was 7.5 ng/mL. sPLA2-GIB levels ranged from 25 pg/mL to 1.3 ng/mL and mean (SD) was 287 (243) pg/mL and the median was 231 pg/mL. Total sPLA2-GIB levels ranged from 2.5 to 20.5 ng/mL, the mean (SD) was 9.3 (3.2) ng/mL and the median was 9.1 ng/mL.

The patient characteristics are presented in Table 6 and the percentile distribution by gender and age, and the mean (SD) of pro-sPLA2-GIB, sPLA2-GIB, and both forms (total) levels are presented in Table 7. As indicated in Table 6, no correlation between pro-sPLA2-GIB, sPLA2-GIB or both forms levels and gender, age or BMI was observed within the tested population.
pro-sPLA2-GIB and total sPLA2-GIB levels were highly correlated (Spearman R = 0.938, p<0.0001), whereas pro-sPLA2 and sPLA2-GIB levels were only weakly correlated (Spearman R = 0.318, p = 0.002).

### In vivo effect of #14G9 mAb on the inhibition of phospho-STAT5 nuclear translocation by sPLA2-IB

The ability of the #14G9 mAb to counterbalance *in vivo* the inhibitory effect of the injection of sPLA2-IB on the IL-7 induced phospho-STAT5 nuclear translocation in CD4 T cells was tested by first injecting 1 mg of the #14G9 mAb in mice, followed by the injection of 100 µg of sPLA2-GIB. Nuclear translocation of phospho-STAT5 in CD4 T cells isolated from splenocytes of the treated mice was then analyzed by confocal microscopy.

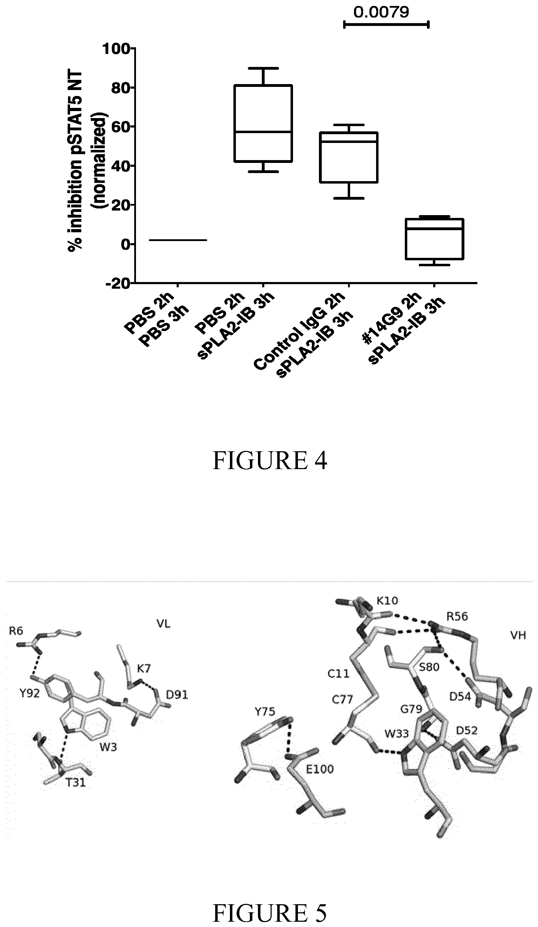

As depicted in Figure 4, the injection of 100 µg of sPLA2-GIB in mice led to a 60% inhibition of the nuclear translocation of phosphoSTAT5 in CD4 T cells isolated from splenocytes. This effect was fully abolished by the prior injection of 1 mg of the #14G9 mAb, with no significant effect observed in control injections.

### Definition of the epitope

The Fab fragment of a humanized form of #14G9 was co-crystallized with its antigen, sPLA2-GIB and x-ray diffraction data of the co-crystal were collected on the PROXIMA-1 Beamline at the Soleil Synchrotron (St. Aubin, France). The crystals belong to the P21 space group (a=71.9Å, b=83.8Å, c=103.9Å; α=β=99.58°) with two sPLA2-GIB/Fab complex per asymmetric unit. The resolution of the structure of the sPLA2-GIB/Fab complex enabled the identification of the critical residues involved in the non-covalent interactions in the complex and definition of the epitope. As presented in the Figure 5, W3, R6, and K7 in the sPLA2-GIB are involved in the interaction with the Fab light chain, and K10, C77, Y75, G79 and S80 in the sPLA2-GIB are involved in the interaction with the Fab heavy chain.

### LIST OF SEQUENCES

| SEQ ID NO: | Object | Sequence |
|---|---|---|
| SEQ ID NO: 1 | PLA2-GIB protein | MKLLVLAVLLTVAAADSGISPRAVWQFRKMIKCVIPGSDPF LEYNNYGCYCGLGGSGTPVDELDKCCQTHDNCYDQAKKLD SCKFLLDNPYTHTYSYSCSGSAITCSSKNKECEAFICNCDRN AAICFSKAPYNKAHKNLDTKKYCQS |
| SEQ ID NO: 2 | 14G9-VL aa | DIVMTQSHKLMSTSVGDRVSITCKASQDVSTAVAWYQEKP GQSPKLLIYWASTRHTGVPNRFTGSRSGTDYTLTISSVQAED LALYYCQQDYSTPPTFGAGTKLELKRADAAPTVSI |
| SEQ ID NO: 3 | 14G9-VL nuc | GACATTGTGATGACCCAGTCTCACAAACTCATGTCCACAT CAGTAGGAGACAGGGTCAGCATCACCTGCAAGGCCAGTC AGGATGTGAGTACTGCTGTAGCCTGGTATCAAGAAAAAC CAGGTCAATCTCCTAAACTACTGATTTACTGGGCATCCAC CCGGCACACTGGAGTCCCTAATCGCTTCACAGGCAGTAG ATCTGGGACAGATTATACTCTCACCATCAGCAGTGTGCAG GCTGAAGACCTGGCACTTTATTACTGTCAGCAAGATTATA GCACTCCTCCTACGTTCGGTGCTGGGACCAAGCTGGAGCT GAAACGGGCTGATGCTGCACCAACTGTATCCATC |
| SEQ ID NO: 4 | 14G9-VH aa | QVQLQQPGAELVKPGASVKLSCKASGYTFTNYWIHWVKRR PGQGLEWIGNIDPSDTRTHYNQKFKDKATLAVDKSSSTAYM QLSSLTSEDSAVFYCARQTLYYEALDYWGQGTTLTVSSAKT TPPSVYPL |
| SEQ ID NO: 5 | 14G9-VH nuc | CAGGTCCAACTGCAACAGCCTGGGGCTGAGCTGGTGAAG CCTGGGGCTTCAGTGAAGCTGTCCTGCAAGGCTTCTGGCT ACACCTTCACCAACTACTGGATACACTGGGTGAAGCGGA GGCCTGGACAAGGCCTTGAATGGATTGGTAATATTGACC CTTCTGATACTAGAACTCACTACAATCAAAAGTTCAAGG ACAAGGCCACATTGGCTGTAGACAAATCCTCCAGCACAG CCTACATGCAGCTCAGCAGCCTGACATCTGAGGACTCTGC GGTCTTTTACTGTGCAAGACAGACCCTCTACTATGAGGCC TTGGACTACTGGGGCCAAGGCACCACTCTCACAGTCTCCT CAGCCAAAACGACACCCCCATCTGTCTATCCACTGGG |
| SEQ ID NO: 6 | 22C6-VL nuc | GAAATTGTTCTCACCCAGTCTCCAACCACCATGGCTGCAT CTCCCGGGGAGAAGATCACTATCACCTGCAGCGTCAGCT CAAGTATAAGTCCCAATTTCTTGCATTGGTATCAGCAGAA GCCAGGATTCTCCCCGAAACTCTTGATTTTTAGGACATCC AATCTGGCTTCTGGAGTCCCAACTCGCTTCAGTGGCAGTG GGTCTGGGACCTCTTACTCTCTCACAATTGGCACCATGGA GGCTGAAGACGTTGCCACTTATTACTGTCACCAGGGTAAT AGTTTACCGCTCACGTTCGGTGCTGGGACCAAGCTGGAG CTGAAACGGGCTGATGCTGCACCAACTGTATCCATCTTCC CACCATCC |
| SEQ ID NO: 7 | 22C6-VL aa | EIVLTQSPTTMAASPGEKITITCSVSSSISPNFLHWYQQKPGFS PKLLIFRTSNLASGVPTRFSGSGSGTSYSLTIGTMEAEDVATY YCHQGNSLPLTFGAGTKLELKRADAAPTVSIFPP |
| SEQ ID NO: 8 | 22C6-VH nuc | GAGGTGCAGCTGCAGGAGTCAGGACCTGGCCTGGTGAAG CCTTCTCAGACAGTGTCCCTCAACTGCACTGTCACTGGCC ACTCTATCACTGATGATAATTACTATTGGAGCTGGATCCG GCTGGTTTCAGGAAGCAAACTGGAGTGGATAGGGTACAT AACCTCCGCTGGTAACACTGACAACAATCCTTCTCTCAAA AGTCGCATCTCCATCACCCGGGACATTTCCAAGAACCAAT TCTTCCTGCACTTGAGCTCTGTGACTGCTGAAGATACAGC CACATATTACTGTGCAAGAGATTCAACTGATGGTGACTTT TACTTTGAAAAATGGGGCCAAGGCACCACTCTCACAGTC TCCTCAGCCAAAACAACAGCCCCATCGGTCTATCCACTGG CCCCTGTGTGTGGAGATACAAGTGGCTCCTCGGTGACTCT AGGATGCCTGGTCAAG |
| SEQ ID NO: 9 | 22C6-VH aa | EVQLQESGPGLVKPSQTVSLNCTVTGHSITDDNYYWSWIRL VSGSKLEWIGYITSAGNTDNNPSLKSRISITRDISKNQFFLHLS SVTAEDTATYYCARDSTDGDFYFEKWGQGTTLTVSSAKTT APSVYPLAPVCGDTSGSSVTLGCLVK |
| SEQ ID NO: 10 | 24B2-VL nuc | GACATTGTGCTGACCCAATCTCCAGCTTCCTTGGCTGTGT CTCTAGGGCAGAGGGCCACCATCTCTTGCAGAGCCAGCG AAAGTGTTGATAATTATGGCATTAGTTTTATGCACTGGTA CCAGCAGAAACCGGGACAGCCACCCAAACTCCTCATCTA TCGTGCATCCAACCTAGAATCTGGGATCCCTGCCAGGTTC AGTGGCAGTGGGTCTAGGACAGACTTCACCCTCACCATT AATCCTGTGGAGACTGATGATGTTGCAACCTATTATTGTC AGCAAAGTAGTAGGGATCTGTTCACGTTCGGAGGGGGGA CTAAGCTGGAAATAAAACGGGCG |
| SEQ ID NO: 11 | 24B2-VL aa | DIVLTQSPASLAVSLGQRATISCRASESVDNYGISFMHWYQQ KPGQPPKLLIYRASNLESGIPARFSGSGSRTDFTLTINPVETD DVATYYCQQSSRDLFTFGGGTKLEIKRA |
| SEQ ID NO: 12 | 24B2-VH nuc | GATGTACAAGTGCAGGAGTCAGGACCTGGCCTGGTGAAG CCTTCTCAGACAGTGTCCCTCAACTGCACTGTCACTGGCC ACTCTATCACTGATGATAATTACTATTGGAGCTGGATCCG GCTGGTTTCTGGAAGCAAATTGGAGTGGATCGGGTACAT AACCTCCAGTGGTAGCACTGACATCAATCCTTCTCTCAAA AGTCGCATCTCCATCACCCGGGACATTTCCAAGAACCAGT TCTTCCTGCACTTGAACTCTGTGACTGCTGAAGATACAGC CACATATTACTGTGCAAGAGATTCAACTGATGGTGACTTT TACTTTGAAAAATGGGGCCAAGGCACCACTCTCACAGTC TCCTCAGCCAAAACAACAGCCCCATCGGTCTATCCACTGG CCCCTGTGTGTGGAGATACA |
| SEQ ID NO: 13 | 24B2-VH aa | DVQVQESGPGLVKPSQTVSLNCTVTGHSITDDNYYWSWIRL VSGSKLEWIGYITSSGSTDINPSLKSRISITRDISKNQFFLHLNS VTAEDTATYYCARDSTDGDFYFEKWGQGTTLTVSSAKTTA PSVYPLAPVCGDT |
| SEQ ID NO: 14 | 8G11-VL nuc | GACATTGTGCTGACACAGTCTCCTGCTTCCTTAGCTGTAT CTCTGGGGCAGAGGGCCACCATCTCATACAGGGCCAGCA AAAGTGTCAGTACATCTGGCTATAGTTATATGCACTGGAA CCAACAGAAACCAGGACAGCCACCCAGACTCCTCATCTA TCTTGTATCCAACCTAGAATCTGGGGTCCCTGCCAGGTTC AGTGGCAGTGGGTCTGGGACAGACTTCACCCTCAACATC CATCCTGTGGAGGAGGAGGATGCTGCAACCTATTACTGT CAGCACATTAGGGAGCTTACACGTTCGGAGGGGGGACCA AGCTGGAAATAAAAC |
| SEQ ID NO: 15 | 8G11-VL aa | DIVLTQSPASLAVSLGQRATISYRASKSVSTSGYSYMHWNQ QKPGQPPRLLIYLVSNLESGVPARFSGSGSGTDFTLNIHPVEE EDAATYYCQHIRELTRSEGGPSWK |
| SEQ ID NO: 16 | 8G11-VH nuc | CAGGTTCAACTGCAGCAGTCTGGGGCTGAGCTGGTGAGG CCTGGGGCTTCAGTGAAGCTGTCCTGCAAGGCTTCGGGCT ACACATTTAATGACGATGAAATGCACTGGGTGAAGCAGA CACCTGTGCATGGCCTGGAATGGATTGGAGCTATTGATCC TGAAACTGGTGGCACTGCCTACAATCAGAAGTTCAAGGA CAAGGCCACACTGACTGCAGACAAATCCTCCAGCACAGC CTACATGGAGATCCGCAGCCTGACATCTGAGGACTCTGC CGTCTATTACTGTCAGTTAATCTTCCATGGTAACCCTTACT ATTGTATGGACTACTGGGGTCAAGGAACCTCAGTCACCG TCTCCTCAGCCAAAACAACAGCCCCATCGGTCTATCCACT GGCCCCTGTGTGTGGAGATACAAGTGGCTCC |
| SEQ ID NO: 17 | 8G11-VH aa | QVQLQQSGAELVRPGASVKLSCKASGYTFNDDEMHWVKQ TPVHGLEWIGAIDPETGGTAYNQKFKDKATLTADKSSSTAY MEIRSLTSEDSAVYYCQLIFHGNPYYCMDYWGQGTSVTVSS AKTTAPSVYPLAPVCGDTSGS |
| SEQ ID NO: 18 | 1C11-VL nuc | GAAATTGTGCTCACTCAGTCTCCAGCCATCACATCTGCAT CTCTGGGGCAAAGGGTCACCATCTCCTGCAGTGCCACCTC AAGTGTAAGTTACGTCCACTGGTACCAGCAGAAGTCAGG CACCTCCCCCAAACCATGGATTTATGAAATATCCAGACTG GCTTCTGGAGTCCCAGCTCGCTTCAGTGGCAGTGGATCTG GGACCTCTTACTCTCTCACAATCAGCACCATGGAGGCTGA AGATGCTGCCATTTATTACTGCCAGTCGTGGAATTTTCCA CTGCTCTCGTTCGGTGCTGGGACCAAGCTGGAGCTGAAA CGGGCTGATGCTGCACCAACTGTATCCATCTTCCCAACCA TCCAA |
| SEQ ID NO: 19 | 1C11-VL aa | EIVLTQSPAITSASLGQRVTISCSATSSVSYVHWYQQKSGTSP KPWIYEISRLASGVPARFSGSGSGTSYSLTISTMEAEDAAIYY CQSWNFPLLSFGAGTKLELKRADAAPTVSIFPTIQ |
| SEQ ID NO: 20 | 1C11-VH nuc | GAGGTGAAGCTGCAGGAGTCAGGACCTGAGCTGGTGAAG CCTGGGGCCTCAGTGAAGATTTCCTGCAAGGTTTCTGGCT ACGCATTCAGTAGCTTCTGGATGAACTGGGTGAAGCAGA GGCCTGGAAAGGGTCTTGAGTGGATTGGACGGATTTATC CTGGAGATGGAGATATTAATTACAATGGGAAGTTCAAGG GCAAGGCCACACTGACCGCAGACAAATCCTCCAGCACAG CCTACATGCAACTCAGCAGCCTGACATCTGAGGACTCTGC GGTCTACTTCTGTGTTAGAGGATGGGCCTGGTTTCCTTAC TGGGGCCAGGGGACTCTAGTCACTGTCTCTGCAGCCAAA ACGACACCCCCATCTGTCTATCCACTG |
| SEQ ID NO: 21 | 1C11-VH aa | EVKLQESGPELVKPGASVKISCKVSGYAFSSFWMNWVKQR PGKGLEWIGRIYPGDGDINYNGKFKGKATLTADKSSSTAYM QLSSLTSEDSAVYFCVRGWAWFPYWGQGTLVTVSAAKTTP PSVYPL |
| SEQ ID NO: 22 | 7A10-VL nuc | GACATCCAAATGAACCAGTCTCCATCCAGTCTGTCTGCAT CCCTCGGAGACACAATTACCATCACTTGCCGTGCCAGTCA GGGCATTAGTATTTGGTTAGACTGGTACCAGCAGAAACC AGGAAATATTCCTAAACTATTGATCTATAAGGCTTCCAAC |
| | | TTGCACACAGGAGTCCCACCAAGGTTTAGTGGCAGTGGA TCTGGAACAGATTTCACATTAACTATCAGCAGCCTACAGC CTGAAGACATTGCCACTTACTACTGTCTACAGAGTCAAAG TTATCCTCTCACGTTCGGTGCTGGGACCAAGCTGGAGCTG AAACGGGCTGATGCTGCACCAACTGTATCCATCTTCCCAC CATCCAAA |
| SEQ ID NO: 23 | 7A10-VL aa | DIQMNQSPSSLSASLGDTITITCRASQGISIWLDWYQQKPGNI PKLLIYKASNLHTGVPPRFSGSGSGTDFTLTISSLQPEDIATY YCLQSQSYPLTFGAGTKLELKRADAAPTVSIFPPSK |
| SEQ ID NO: 24 | 7A10-VH nuc | CAGGTGCAGCTGAAGGAGTCAGGACCTGGCCTGGTGGCG CCCTCACAGAGCCTGTCCATCACATGCACTGTCTCAGGGT TCTCATTAACCAGCTATGGTGTAAGCTGGGTTCGCCAGCC TCCAGGAAAGGGTCTGGAGTGGCTGGGAGTAATATGGGG TGACGGGAGCACAAATTATCATTCAGCTCTCATATCCAGA CTGAGCATCAGCAAGGATAACTCCAAGAGCCAAGTTTTC TTAAAACTGAACAGTCTGCAAACTGATGACACAGCCACG TACTACTGTGCCAAAGAAGGAGGATTACGACGCGGGGTC TACTTTGACTACTGGGGCCAAGGCACCACTCTCACAGTCT CCTCAGCCAAAACGACACCCCCATCTGTCAATTCCACTGA A |
| SEQ ID NO: 25 | 7A10-VH aa | QVQLKESGPGLVAPSQSLSITCTVSGFSLTSYGVSWVRQPPG KGLEWLGVIWGDGSTNYHSALISRLSISKDNSKSQVFLKLNS LQTDDTATYYCAKEGGLRRGVYFDYWGQGTTLTVSSAKTT PPSVNSTE |
| SEQ ID NO: 26 | sPLA2-GIB protein | AVWQFRKMIKCVIPGSDPFLEYNNYGCYCGLGGSGTPVDEL DKCCQTHDNCYDQAKKLDSCKFLLDNPYTHTYSYSCSGSAI TCSSKNKECEAFICNCDRNAAICFSKAPYNKAHKNLDTKKY CQS |

**Table 1**

| **Subclone** | **Antigen used for Immunization** | **Isotype** | **Enzymatic Activity Inhibition (% of Control)** | **pro-sPLA2-IB ELISA EC50 (nM)** | **sPLA2-IB ELISA EC50 (nM)** |
|---|---|---|---|---|---|
| **#8E7** | sPLA2-IB | IgG1 | 29% | 151 | 269 |
| **#9H5** | sPLA2-IB | IgG1 | 107% | 311 | 253 |
| **#6F7** | sPLA2-IB | IgG2a | 104% | 2 | 3 |
| **#14G9** | sPLA2-IB | IgG1 | 5% | 158 | 6 |
| **#20F4** | sPLA2-IB | IgG1 | 32% | 10 | 13 |
| **#4G11** | sPLA2-IB | IgG1 | 27% | 2 | 3 |
| **#3F11** | sPLA2-IB | IgG1 | 42% | 13 | 20 |
| **#7A10** | sPLA2-IB | IgG1 | 51% | 13 | 77 |
| #1C11 | sPLA2-IB | IgG1 | 110% | 4 | 3 |
| **#34G3** | sPLA2-IB | IgG2a | 5% | 8 | 10 |
| **#22H5** | sPLA2-IB | IgG2a | 12% | 30 | 29 |
| **#36G10** | sPLA2-IB | IgG2a | 19% | 12 | 8 |
| **#22C6** | sPLA2-IB | IgG2a | 3% | 117 | 22 |
| **#5G7** | sPLA2-IB | IgG1 | 0% | 56 | 61 |
| **#23C7** | sPLA2-IB | IgG2a | 0% | 69 | 14 |
| **#23F10** | sPLA2-IB | IgG2a | 0% | 66 | 18 |
| **#24B2** | sPLA2-IB | IgG2a | 5% | 81 | 15 |
| **#28A1** | sPLA2-IB | IgG2a | 3% | 61 | 11 |
| **#28A3** | sPLA2-IB | IgG2a | 7% | 77 | 11 |
| **#36G5** | sPLA2-IB | IgG2a | 0% | 147 | 22 |
| **#8G11** | N-ter peptide | IgG2a | 112% | 6 | 162 |
| **#18C8** | N-ter peptide | IgG1 | 111% | 91 | 70 |
| **#3G5** | N-ter peptide | IgG1 | 56% | 23 | 3200 |
| **#3B2** | pro-sPLA2-IB | IgG1 | 92% | 24 | 46 |
| **#4E2** | pro-sPLA2-IB | IgG2b | 96% | 39 | 47 |
| **#18D9** | pro-sPLA2-IB | IgG2b | 90% | 213 | 293 |
| **#7H12** | pro-sPLA2-IB | IgG2a | 95% | 3 | 3 |
| **#6G11** | pro-sPLA2-IB | IgG1 | 97% | 34 | 18 |
| **#14G2** | pro-sPLA2-IB | IgG2a | 57% | 2 | 1 |
| **#7E2** | pro-sPLA2-IB | IgG2a | 62% | 3 | 3 |
| **#17A7** | pro-sPLA2-IB | IgG2a | 140% | 2 | 2 |
| **#18A6** | pro-sPLA2-IB | IgG2a | 24% | 9 | 8 |
| **#7C5** | pro-sPLA2-IB | IgG2a | 168% | 3 | 3 |
| **#15C1** | pro-sPLA2-IB | IgG2a | 56% | 4 | 4 |
| **#15E12** | pro-sPLA2-IB | IgG1 | 54% | 80 | 79 |
| **#13H8** | pro-sPLA2-IB | IgG2b | 96% | 33 | 41 |

**Table 2**

| Subclone | pro-sPLA2-IB IC50 (nM) | sPLA2-IB IC50 (nM) |
|---|---|---|
| #7A10 | 4.3 | 5.3 |
| #14G9 | 108.6 | 1.0 |
| #1C11 | 2.4 | 3.8 |
| #6F7 | 13.9 | 8.2 |
| #4G11 | 1.7 | 0.7 |
| #8G11 | 8.4 | N/A |

**Table 3**

| mAb | Antigen | kₒₙ (10⁴ M⁻¹.s⁻¹) | k_{off} (10⁻⁴ s⁻¹) | t _{1/2} (min) | K_{d} (pM) |
|---|---|---|---|---|---|
| #14G9 | sPLA2-GIB | 163 ± 11 | 5.49 ± 0.23 | 30.4 ± 1.3 | 338 ± 10 |
| #14G9 | pro-sPLA2-GIB | 30.1 ± 1.9 | 506 ± 30 | 0.30 ± 0.02 | 174000 ± 7000 |
| #1C11 | sPLA2-GIB | 479 ± 17 | 3.09 ± 0.06 | 53.9 ± 1.0 | 64.4 ± 1.1 |
| #1C11 | pro-sPLA2-GIB | 270 ± 37 | 4.05 ± 0.37 | 41.2 ± 3.8 | 151 ± 12 |
| #7A10 | sPLA2-GIB | 11.8 ± 1.7 | 4.44 ± 0.05 | 37.5 ± 0.4 | 3830 ± 590 |
| #7A10 | pro-sPLA2-GIB | 11.0 ± 1.4 | 4.07 ± 0.12 | 41.0 ± 1.2 | 3790 ± 540 |
| #8G11 | sPLA2-GIB | n.d. | n.d. | n.d. | > 10⁷ (10µM) |
| #8G11 | pro-sPLA2-GIB | 2.06 ± 0.14 | 7.03 ± 0.06 | 23.7 ± 0.2 | 34300 ± 2700 |

| | | | | | |
|---|---|---|---|---|---|
| n.d. : non detectable. t _{½}: half-live constant (= 1/ k_{off}). | | | | | |

**Table 4**

| Biotinylated mAb | 1C11 | | 7A10 | | 8G11 | 14G9 |
|---|---|---|---|---|---|---|
| Coated Antigen | pro-sPLA2--IB | sPLA2-IB | pro-sPLA2-IB | sPLA2-IB | pro-sPLA2-IB | sPLA2-IB |
| Competing mAb | | | | | | |
| 8E7 | 38% | 34% | 142% | 151% | 87% | 107% |
| 9H5 | 105% | 156% | 113% | 220% | 120% | 302% |
| 6F7 | 98% | 106% | 63% | 270% | 303% | 262% |
| 14G9 | 69% | 64% | 141% | 12% | 73% | 0% |
| 20F4 | 101% | 167% | 281% | 628% | 228% | 899% |
| 4G11 | 13% | 33% | 102% | 534% | 408% | 808% |
| 1C11 | 0% | 0% | 314% | 1146% | 496% | 1441% |
| 3F11 | 156% | 181% | 155% | 336% | 123% | 367% |
| 7A10 | 18% | 19% | 0% | 0% | 93% | 58% |
| 34G3 | 13% | 58% | 137% | 22% | 65% | 10% |
| 22H5 | 16% | 33% | 127% | 94% | 69% | 19% |
| 36G10 | 26% | 58% | 125% | 21% | 50% | 46% |
| 23F10 | 20% | 58% | 135% | 44% | 20% | 45% |
| 36G5 | 21% | 57% | 156% | 128% | 31% | 47% |
| 5G7 | 37% | 56% | 87% | 35% | 41% | 102% |
| 23C7 | 23% | 65% | 134% | 58% | 17% | 32% |
| 24B2 | 23% | 61% | 135% | 65% | 64% | 17% |
| 22C6 | 18% | 56% | 141% | 53% | 39% | 2% |
| 28A1 | 24% | 50% | 124% | 69% | 17% | 7% |
| 28A3 | 32% | 59% | 123% | 64% | 29% | 8% |
| 8G11 | 14% | 87% | 103% | 12% | 0% | 43% |
| 18C8 | 54% | 86% | 55% | 48% | 28% | 36% |
| 3B2 | 30% | 46% | 10% | -55% | 70% | 63% |
| 4E2 | 47% | 96% | 51% | 42% | 56% | 69% |
| 18D9 | 47% | 94% | 53% | 72% | 12% | 113% |
| 7H12 | 23% | 36% | 170% | 645% | 1282% | 68% |
| 6G11 | 83% | 99% | 74% | 78% | 89% | 97% |
| 14G2 | 39% | 48% | 457% | 1040% | 1530% | 1317% |
| 7E2 | 24% | 27% | 146% | 585% | 465% | 609% |
| 17A7 | 54% | 104% | 152% | 454% | 2743% | 34% |
| 18A6 | 39% | 52% | 68% | -7% | 229% | 28% |
| 7E5 | 54% | 81% | 41% | 109% | 1361% | 17% |
| 15C1 | 42% | 55% | 354% | 1164% | 1992% | 1059% |
| 15E12 | 88% | 118% | 58% | 64% | 408% | 342% |
| 13H8 | 22% | 72% | 6% | -81% | 36% | 28% |
| 3G5 | 60% | 76% | 59% | 97% | 48% | 50% |

**Table 5**

| | #8G11/#1C11 ELISA | #14G9/#1C11 ELISA | #7A10/#1C11 ELISA |
|---|---|---|---|
| Accuracy (% Recovery) | between 90% and 92% | between 85% and 103% | between 83% and 91% |
| LD | 0.10 ng/mL | 0.02 ng/mL | 0.10 ng/mL |
| LLOQ | 0.04 ng/mL | 0.02 ng/mL | 0.08 ng/mL |
| HLOQ | 5 ng/mL | 1 ng/mL | 5 ng/mL |
| Intra-run precision (%CV) | between 4.0% and 14.2% | between 3.2% and 9.3% | between 5.1% and 10.2% |
| Inter-run (%CV) precision | between 14.5% 2.3% and | between 18.8% 6.4% and | between 12.3% 6.7% and |
| Linearity (% Recovery) | between 111% 108% and | between 104% 100% and | between 86% and 91% |

**Table 6**

| | | Median | | Median | | | Median | |
|---|---|---|---|---|---|---|---|---|
| | n | pro-sPLA2-IB (ng/mL) | p value | total (ng/mL) | sPLA2-IB | p value | sPLA2-IB (pg/mL) | P value |
| All patients | 99 | 7.5 | | 9.1 | | | 231 | |
| Gender | | | | | | | | |
| Male | 50 | 7.2 | | 9.3 | | | 278 | |
| Female | 49 | 8.1 | | 8.7 | | | 197 | |
| Age (years) | | | 0.158 | | | 0.142 | | -0.039 |
| 25-29 | 12 | 7.9 | | 8.9 | | | 185 | |
| 30-39 | 36 | 6.9 | | 9.0 | | | 240 | |
| 40-49 | 25 | 7.5 | | 9.3 | | | 296 | |
| 50-59 | 26 | 7.9 | | 9.4 | | | 207 | |
| BMI (kg/m2) | | | -0.167 | | | -0.183 | | -0.068 |
| <25 | 19 | 7.7 | | 8.9 | | | 231 | |
| 25-30 | 37 | 8.2 | | 10.0 | | | 274 | |
| 30-40 | 30 | 6.7 | | 8.0 | | | 224 | |
| >40 | 13 | 7.1 | | 8.1 | | | 198 | |

**Table 7**

| Percentile | | | | | | | Mean | SD |
|---|---|---|---|---|---|---|---|---|
| | n | 10% | 25% | 50% | 75% | 90% | | |
| **ALL** | | | | | | | | |
| pro-sPLA2-IB (ng/mL) | 99 | 4.5 | 6.1 | 7.5 | 9.6 | 11.3 | 7.8 | 2.8 |
| total sPLA2-IB (ng/mL) | 99 | 5.6 | 7.2 | 9.1 | 11.2 | 13.6 | 9.3 | 3.2 |
| sPLA2-IB (pg/mL) | 97 | 82 | 137 | 231 | 325 | 520 | 287 | 243 |

| **MALE** | | | | | | | | |
|---|---|---|---|---|---|---|---|---|
| pro-sPLA2-IB (ng/mL) | 50 | 4.0 | 6.0 | 7.2 | 9.6 | 11.9 | 7.5 | 2.8 |
| total sPLA2-IB (ng/mL) | 50 | 5.4 | 7.1 | 9.3 | 11.2 | 13.8 | 9.2 | 3.0 |
| sPLA2-IB (pg/mL) | 48 | 115 | 182 | 278 | 336 | 602 | 328 | 265 |

| **FEMALE** | | | | | | | | |
|---|---|---|---|---|---|---|---|---|
| pro-sPLA2-IB (ng/mL) | 49 | 4.7 | 6.1 | 8.1 | 9.6 | 11.3 | 8.1 | 2.8 |
| total sPLA2-IB (ng/mL) | 49 | 5.8 | 7.2 | 8.7 | 11.2 | 13.6 | 9.5 | 3.4 |
| sPLA2-IB (pg/mL) | 49 | 57 | 113 | 197 | 321 | 499 | 247 | 214 |

| **25-29 years old** | | | | | | | | |
|---|---|---|---|---|---|---|---|---|
| pro-sPLA2-IB (ng/mL) | 12 | 4.2 | 5.5 | 7.9 | 8.7 | 14.0 | 7.8 | 3.1 |
| total sPLA2-IB (ng/mL) | 12 | 5.0 | 6.5 | 8.9 | 10.3 | 15.3 | 9.0 | 3.2 |
| sPLA2-IB (pg/mL) | 12 | 76 | 157 | 185 | 309 | 375 | 217 | 98 |

| **30-39 years old** | | | | | | | | |
|---|---|---|---|---|---|---|---|---|
| pro-sPLA2-IB (ng/mL) | 36 | 3.5 | 5.0 | 6.9 | 9.3 | 10.8 | 7.2 | 3.1 |
| total sPLA2-IB (ng/mL) | 36 | 4.2 | 6.2 | 9.0 | 10.9 | 13.4 | 8.9 | 3.7 |
| sPLA2-IB (pg/mL) | 34 | 70 | 140 | 240 | 348 | 559 | 310 | 288 |

| **40-49 years old** | | | | | | | | |
|---|---|---|---|---|---|---|---|---|
| pro-sPLA2-IB (ng/mL) | 25 | 4.8 | 6.0 | 7.5 | 9.7 | 12.3 | 7.9 | 2.5 |
| total sPLA2-IB (ng/mL) | 25 | 6.1 | 7.2 | 9.3 | 11.6 | 14.4 | 9.6 | 2.9 |
| sPLA2-IB (pg/mL) | 25 | 65 | 137 | 296 | 517 | 812 | 354 | 305 |

| 50-59 years old | | | | | | | | |
|---|---|---|---|---|---|---|---|---|
| pro-sPLA2-IB (ng/mL) | 26 | 6.0 | 6.6 | 7.9 | 10.4 | 12.3 | 8.5 | 2.4 |
| total sPLA2-IB (ng/mL) | 26 | 6.9 | 7.8 | 9.4 | 11.6 | 14.0 | 9.8 | 2.7 |
| sPLA2-IB (pg/mL) | 26 | 99 | 132 | 207 | 305 | 380 | 225 | 110 |

## Claims

**1.** A monoclonal antibody, or a variant or fragment thereof, that binds to human sPLA2-GIB protein, wherein said antibody, variant or fragment competitively inhibits the binding of monoclonal antibody 14G9 to human sPLA2-GIB, said monoclonal antibody 14G9 comprising a light chain variable region comprising SEQ ID NO: 2 and a heavy chain variable region comprising SEQ ID NO: 4.

**2.** A monoclonal antibody or fragment thereof according to claim 1, wherein said antibody inhibits sPLA2-GIB.

**3.** A monoclonal antibody or fragment thereof according to claim 1, wherein said antibody binds an epitope comprising at least one amino acid residue selected from W3, R6, K7, K10, C77, Y75, G79 and S80 of human sPLA2-GIB protein, by reference to SEQ ID NO: 26.

**4.** A monoclonal antibody or fragment thereof that binds to an epitope on human sPLA2-GIB protein, wherein said epitope comprises at least one amino acid residue selected from W3, R6, K7, K10, C77, Y75, G79 and S80 of human sPLA2-GIB protein.

**5.** A monoclonal antibody or fragment thereof according to claim 1, wherein said antibody comprises a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 2 and a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 4.

**6.** A monoclonal antibody or fragment thereof according to claim 1, wherein said antibody comprises a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 5 and a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 7.

**7.** A monoclonal antibody or fragment thereof according to claim 1, wherein said antibody comprises a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 11 and a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 13.

**8.** A monoclonal antibody or fragment thereof that binds to human pro-PLA2-GIB protein, wherein said antibody competitively inhibits the binding of monoclonal antibody 8G11 to human pro-PLA2-GIB, said monoclonal antibody 8G11 comprising a light chain variable region comprising SEQ ID NO: 15 and a heavy chain variable region comprising SEQ ID NO: 17.

**9.** A monoclonal antibody or fragment thereof according to claim 8, wherein said antibody comprises a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 15 and a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 17.

**10.** A monoclonal antibody or fragment thereof that binds both the secreted form (sPLA2-GIB) and the pro- form (pro-PLA2-GIB) of human PLA2-GIB protein.

**11.** A monoclonal antibody or fragment thereof according to claim 11, wherein said antibody competitively inhibits the binding of monoclonal antibody 1C11 to secreted and/or pro- form of human PLA2-GIB protein, said monoclonal antibody 1C11 comprising a light chain variable region comprising SEQ ID NO: 19 and a heavy chain variable region comprising SEQ ID NO: 21.

**12.** A monoclonal antibody or fragment thereof according to claim 11, wherein said antibody comprises a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 19 and a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 21.

**13.** A monoclonal antibody or fragment thereof according to claim 11, wherein said antibody competitively inhibits the binding of monoclonal antibody 7A10 to secreted and/or pro- form of human PLA2-GIB protein, said monoclonal antibody 7A10 comprising a light chain variable region comprising SEQ ID NO: 23 and a heavy chain variable region comprising SEQ ID NO: 25.

**14.** A monoclonal antibody or fragment thereof according to claim 13, wherein said antibody comprises a light chain variable region comprising a CDR-L1, a CDR-L2 and/or a CDR-L3 consisting, or consisting essentially, of CDR-L1, CDR-L2 and CDR-L3, respectively, of the light chain variable region of SEQ ID NO: 23 and a heavy chain variable region comprising a CDR-H1, a CDR-H2 and/or a CDR-H3 consisting, or consisting essentially, of CDR-H1, CDR-H2 and CDR-H3, respectively, of the heavy chain variable region of SEQ ID NO: 25.

**15.** A composition comprising an antibody of anyone of claims 1-14.

**16.** A nucleic acid encoding an antibody of anyone of claims 1-14, or a light or heavy chain thereof, or a variable domain thereof.

**17.** A method for detecting human PLA2-GIB protein in a sample, comprising:
(i) contacting the sample with a monoclonal antibody of anyone of claims 1 to 14, and
(ii) detecting the presence of an antibody:antigen complex formed in step (i).

**17.** A method according to claim 16, wherein the sample is divided in replicates to detect separately the mature secreted form (sPLA2-GIB), the pro- form (pro-PLA2-GIB) and/or both forms of the human PLA2-GIB protein using any combination of antibodies of anyone of claims 1 to 14.

**18.** A method according to claim 16 or 17 for dosing or monitoring the amount of the mature secreted form (sPLA2-GIB), the pro- form (pro-PLA2-GIB) and/or the total human PLA2-GIB protein.

**19.** A kit comprising a least one antibody or fragment thereof according to anyone of claims 1-14 and a reagent to perform, detect or quantify an immune reaction, or an antibody-antigen complex.
